# EUROPEAN PATENT SPECIFICATION

(11) **EP 1 166 745 B1**
(45) Date of publication and mention of the grant of the patent: **07.11.2007**
(21) Application number: 00912994.1
(22) Date of filing: 30.03.2000
(51) Int. Cl.: A61K 8/73, A61Q 5/12, A61Q 19/00, A61Q 1/00

(54) **COSMETICS CONTAINING POLYSACCHARIDE-STEROL DERIVATIVES**
KOSMETISCHE ZUSAMMENSETZUNGEN ENTHALTEND POLYSACCHARID-STEROL-DERIVATE
PRODUITS COSMETIQUES CONTENANT DES DERIVES DE POLYSACCHARIDE-STEROL

(30) Priority: 31.03.1999 JP 9240199
(43) Date of publication of application: 02.01.2002
(73) Proprietor: NOF CORPORATION, Tokyo 150-6019 (JP); Sunamoto, Junzo, Kusatsu-shi, Shiga 525-0026 (JP)
(72) Inventor: SUNAMOTO, Junzo, Kusatsu-shi Shiga 525-0026 (JP); SHIMADA, Kunio, Toride-shi Ibaraki 302-0012 (JP); HAYASHI, Akio, Tokyo 121-0055 (JP); HOSOTANI, Ryuzo, Hyogo 663-8132 (JP); YANO, Yoshihiro, Tsukuba-shi Ibaraki 305-0821 (JP); AKIYOSHI, Kazunari, Uji-shi Kyoto 611-0042 (JP)
(74) Representative: Bannerman, David Gardner
(86) International application number: PCT/JP2000/002044
(87) International publication number: WO 2000/057841

(56) References cited:
- EP-A- 0 370 810
- EP-A- 1 113 023
- EP-A1- 0 370 810
- WO-A1-00/12564
- JP-A- 3 292 301
- JP-A- 9 095 418
- JP-A- 9 188 604
- JP-A- 10 231 229
- JP-A- 63 319 046
- US-A- 5 372 814
- PATENT ABSTRACTS OF JAPAN vol. 016, no. 126 (C-0923), 31 March 1992 (1992-03-31) & JP 03 292301 A (NIPPON OIL & FATS CO LTD; others: 01), 24 December 1991 (1991-12-24)
- DATABASE WPI Section Ch, Week 198906 Derwent Publications Ltd., London, GB; Class B07, AN 1989-044687 XP002328767 & JP 63 319046 A (EISAI CO LTD) 27 December 1988 (1988-12-27)
- DATABASE WPI Section Ch, Week 199345 Derwent Publications Ltd., London, GB; Class B05, AN 1993-357133 XP002328768 & JP 05 262645 A (NIPPON OILS & FATS CO LTD) 12 October 1993 (1993-10-12)
- DATABASE WPI Section Ch, Week 199327 Derwent Publications Ltd., London, GB; Class B01, AN 1993-216654 XP002328769 & JP 05 139967 A (NIPPON OILS & FATS CO LTD) 8 June 1993 (1993-06-08)
- DATABASE WPI Section Ch, Week 198621 Derwent Publications Ltd., London, GB; Class A96, AN 1986-133838 XP002328770 & JP 61 069801 A (SUNAMOTO J) 10 April 1986 (1986-04-10)
- DATABASE WPI Section Ch, Week 199235 Derwent Publications Ltd., London, GB; Class A11, AN 1992-287035 XP002328771 & JP 03 292301 A (NIPPON OILS & FATS CO LTD) 24 December 1991 (1991-12-24)

## Description

The present invention relates to the use in a cosmetic product of a pullulan-cholesterol derivative. More specifically, the invention relates to the use in a cosmetic product of a pullulan-cholesterol derivative, which is superior in its moisture-retaining effect on skin and in an effect of improving skin roughness, on the one hand, and is superior in the protective effect on hair as a result of a film-forming function, on the other hand, together with superior product stability.

In general, drying of skin occurs when the barrier function of keratin becomes decreased and transcutaneous moisture transpiration becomes increased due to reduction of amount of skin secretory substances, in particular, sebum secretion, as well as due to decrease in the natural moisture-retaining factors, such as intercellular lipids and amino acids. Therefore, drying of skin occurs increasingly by decrease in skin secretory substances in winter season or due to, for example, excessive skin washing, aging and physical constitution, wherein, in particular, a state in which the keratin moisture decreases below 10 % by weight is called dry skin. When skin becomes dry, its appearance will be dull and fine wrinkles become undesirably conspicuous. Also hair loses its smoothness and decreases in lustre undesirably due to a decrease in its moisture content.

In order to improve such conditions of skin and hair, it is necessary to prevent reduction of moisture content in keratin and in hair to maintain their normal function, wherefor moisture retaining agents have hitherto been examined. As a result, it has been confirmed that it is effective for increasing moisture retention to incorporate a polyhydric alcohol exhibiting better adhesion onto skin and having a hydrophobic function, such as vaseline or glycerin; a polysaccharide, such as hyaluronic acid, sorbitol or pullulan; sodium lactate; or an amino acid, such as sodium pyrrolidonecarboxylate. Recently, it has been practised also to incorporate a sphingolipid and ceramide which are constituents of the intercellular lipids in keratin.

However, such preparations known hitherto are not sufficient in moisture-retaining bility and, in addition, they reveal a defect that an unpleasant adherent touch or feel due to oily consistency may be brought about when a blocking agent is incorporated, and, on the other hand, the moisture retaining agent has to be incorporated at a higher proportion, giving rise to an adherent and moistening feel. Moreover, they are defective in durable stability and in stability towards microorganisms.

There are already known cosmetic products containing polysaccharides or pullulan derivatives see for example the following Japanese Patent Kokai publications:
(1) Japanese Patent Kokai Sho 53-142540 A discloses a cosmetic oil-soluble finishing product providing for superior hand feel without causing skin irritation, which contains a fatty acid pullulan ester.
(2) Japanese Patent Kokai Sho 63-66107 A discloses oil/water or water/oil emulsion type cosmetic products formulated with the incorporation of pullulan.
(3) Japanese Patent Kokai Sho 63-139105 A discloses a wrinkle-smoothing cosmetic product characterized by incorporating pullulan.
(4) Japanese Patent Kokai Hei 2-42011 A discloses a cosmetic make-up product in the form of a film of a thickness of 0.01-1 mm characterized by a content of pullulan.
(5) Japanese Patent Kokai Hei 10-182341 A discloses a cosmetic pack containing a fatty acid pullulan ester.

However, conventional cosmetic products such as given above do not include any having a content of a pullulan-cholesterol derivative.

While, in addition, polysaccharide-sterol derivatives have been disclosed in Japanese Patent Kokais Hei 3-292301 A, Hei 5-262645 A and Sho 63-319046A, it has not been known to use them in cosmetic products.

JP 03292301 teaches that polysaccharide-sterol derivatives can be used as a coating material for liposomes, or as a carrier for a medicament, but does not disclose that the derivative may be used in a cosmetic product. EP 370810 discloses a pullulan-cholesterol derivative including a spacer for bonding the pullulan and cholesterol components having a structure of -OCH₃CONHCH₃CH₃NH-R, where R represents cholesterol. In addition, EP 370810 describes a fatty emulsion which is to be used for medicaments and for foodstuffs, and not cosmetic components. JP 63319046 discloses a liquid emulsion for use in medicaments without mentioning any possible cosmetic use.

JP 05262645 teaches a lipid oil-in-water emulsion for an anti-tumour drug comprising α-linolenic acid and a fatty acid triglyceride and a polysaccharide-cholesterol derivative which is said to be effective in reducing the size of tumours in mice, but no suggestion of its use in a cosmetic product is given. JP 05139967 teaches a fatty emulsion for cancer therapeutics containing a pullulan-cholesterol derivative, α-linolenic acid and water, with no mention of any use in a cosmetic product. JP . 61069801 describes cholesterol derivatives of, for example, pullulan, mannan, etc., which are said to be useful as materials for medical uses, but without mentioning cosmetic uses.

The object of the present invention is to provide a cosmetic product which is superior in moisture retaining ability and in film-forming ability to attain thereby improvement of the condition of skin or hair caused by drying, such as rough skin and defective luster, and to provide a wet state of so-called beautiful skin or beautiful hair by retaining sufficient moisture, together with superior touch/feel.

The inventors have found that a cosmetic product having such effects that moisture retaining ability is high due to hygroscopic and moisture-retaining function, that facilitating lamella formation and stabilization are superior and that film-forming ability is superior and an oily feel on the skin is suppressed, when a pullulan-cholesterol derivative obtained by reacting a pullulan which is superior in film-forming ability with a cholesterol which is found in the intercellular lipids is incorporated in a cosmetic product.

The invention is defined in the accompanying claim 1.

The proportion of introduction of the cholesteryl groups is preferably 0.05-15 groups per 100 mono-saccharide units constituting the pullulan.

The proportion of introduction of the cholesteryl groups is preferably 0.1 - 10 groups per 100 monosaccharide units constituting the pullulan.

The content of the pullulan-cholesterol derivative is preferably in the range from 0.001 to 50 %, based on the total weight of the cosmetic product.

The cosmetic product may be a skin-care cosmetic, make-up cosmetic or hair conditioning cosmetic, or an emulsion, a beauty wash, a rouge, a manicure or a hair lotion.

The pullulan-cholesterol derivative can be synthesized by reacting a compound having at one terminal end of the molecule a cholesteryl group and at the other terminal end of the molecule an isocyanato group with a hydroxyl group on the pullulan.

The compound having at one terminal end of the molecule a cholesteryl group and at the other terminal end of the molecule an isocyanato group is obtained, for example, by reacting one isocyanato group at one end of a diisocyanate with a hydroxyl group on a cholesterol molecule to bind them via a urethane bond, as shown by the following reaction scheme (2):

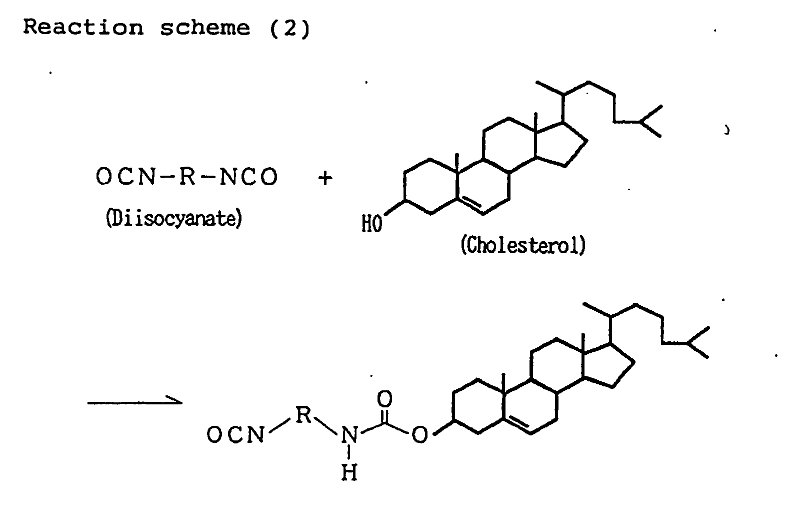

The diisocyanate to be reacted with the sterol is a compound represented by O C N - R - N C O. There may be enumerated, for example, ethylene diisocyanate in which the group R stands for ethylene, butylene diisocyanate in which R stands for butylene, hexamethylene diisocyanate in which R stands for hexamethylene and diphenylmethane diisocyanate in which R stands for diphenylmethane, wherein preference is given to butylene diisocyanate and hexamethylene diisocyanate.

The pullulan-cholesterol derivatives to be used favorably according to the present invention can be obtained by reacting a compound having a cholesteryl group at one terminal end of the molecule and an isocyanato group at the other terminal end with the pullulan. The reaction of the compound having a cholesteryl group and an isocyanato group at one terminal end and at the other terminal end of the molecule, respectively, with the pullulan may be realized in a one-step addition reaction of a hydroxyl group on the monosaccharide constituting the pullulan with the isocyanato group of the compound having a cholesteryl group and an isocyanato group at one terminal end and at the other terminal end of the molecule, respectively, as shown, for example, by the following reaction scheme (3):

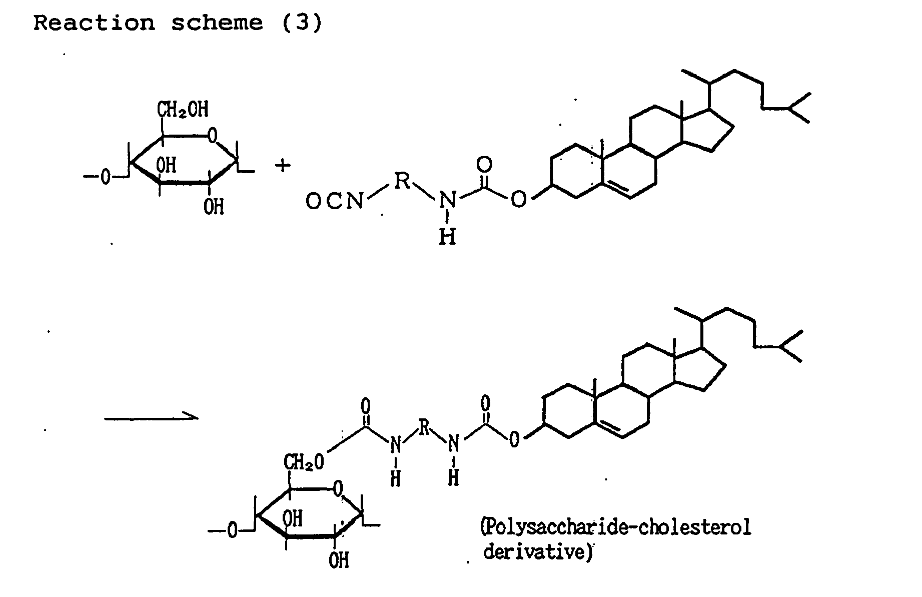

While the reaction scheme (3) shows a reaction of a hexose unit with an isocyanato-containing compound as a reaction model, it is favorable in the synthesis of a pullulan-cholesterol derivative according to the present invention to cause the reaction as indicated by the reaction scheme (3) for the hydroxyl groups on the monosaccharide units constituting the pullulan in a proportion of 0.01 to 20 groups, preferably 0.05 to 15 groups, more preferably 0.1 to 10 groups, per 100 monosaccharide units, in view of increasing the realistic application feel as a cosmetic.
Pullulan is easily available, and has a better adherent touch or feel when incorporated in cosmetic products.

As the solvent to be used on the reaction of the compound having a cholesteryl group at one terminal end and an isocyanato group at the other terminal end of the molecule with the pullulan, there may favorably be used those in which both the compound having a cholesteryl group at one terminal end and an isocyanato group at the other terminal end of the molecule and the pullulan are soluble and the reaction product, i.e. the pullulan-cholesterol derivative, is also soluble therein, wherein, in general, an aprotic solvent, such as dimethylformamide, dimethyl sulfoxide, formamide, dioxane and tetrahydrofuran, may favorably be employed. While the reaction temperature and the reaction duration may adequately be selected in accordance with the solvent and so on used and in dependence on the condition of progress of the reaction, it is preferable to effect the reaction at a temperature in the range from 0 to 200 °C for a duration of about 1 - 48 hours.

The charging amounts of the pullulan and of the compound having a cholesteryl group at one terminal end and an isocyanato group at the other terminal end of the molecule may be in any proportion, wherein the rate of introduction of the cholesteryl group into the pullulan can pertinently be controlled by varying the charging ratio. It is favorable for introducing the cholesteryl group into the pullulan at a proportion of 0.01 - 20 cholesteryl groups per 100 monosaccharide units constituting the pullulan that the compound having a cholesteryl group at one terminal end and an isocyanato group at the other terminal end of the molecule is charged in an amount corresponding to a proportion of 0.01 - 30 molecules of the compound per 100 monosaccharide units constituting the pullulan.

For purifying the resulting pullulan-cholesterol derivative, purification techniques including re-precipitation, chromatographic separation and dialysis may favorably be employed. For realizing drying, there may favorably be employed freeze-drying and vacuum drying.

The pullulan-cholesterol derivative to be used in the cosmetic product according to the present invention may be not only those which are produced by the process described above but also those which are produced by any other voluntary process. In such cases, it is preferable to use a pullulan-cholesterol derivative in which the cholesteryl group is introduced into the pullulan at a proportion of 0.05 - 15 cholesteryl groups, more preferably 0.1- 10 cholesteryl groups, per 100 monosaccharide units constituting the pullulan, since it is superior not only in the ability of retaining water due to the hygroscopic and moisture-preserving functions but also in the function of facilitating and stabilizing lamella formation together with superior film-forming ability.

While there is no special limitation in the molecular weight of the pullulan-cholesterol derivative according to the present invention, it is favorable to use, for cosmetic products for skin care, those which have weight-average molecular weights in the range from 10,000 to 500,000, preferably from 30,000 to 300,000, since they are superior in the effect of improving the moisture preservation and rough skin prevention. For the cosmetic products for hair care, those which have weight-average molecular weight in the range from 10,000 to 500,000, preferably from 30,000 to 300,000, are preferred, since they are superior in the protection effect due to the film-forming ability and in the stability of the product. For the products for rouge, those which have weight-average molecular weights in the range from 10,000 to 1,000,000, preferably from 30,000 to 500,000, are preferred.

The cosmetic products according to the present invention comprise the pullulan-cholesterol derivative and cosmetic ingredients, wherein there is no restriction in their kinds, forms and so on and any product of any kind and form known from the past may be used. Concrete examples of the cosmetic products include skin-care cosmetics, make-up cosmetics and hair conditioning cosmetics.

As the materials for the cosmetic product to be used according to the present invention, known materials for cosmetic products compounded in conventional cosmetic products can be used without any restriction under a pertinent selection in accordance with each specific cosmetic product contemplated. Concrete examples of the materials for the cosmetic products include oils, moisture-preserving agent, UV absorber, beauty whitening agent, moisture-preserving agent, antioxidant, antiseptic, powder, pearly-lustering agent, inorganic pigments, organic pigments, dyestuffs, coloring agents, surfactants, thickening agent, stabilizers, dispersant, antiseptic, sterilizer, thickening agent, plasticizer, medicaments, flagrances, resins, water and pH regulator.

The cosmetic product according to the present invention may favorably contain the cosmetic materials in a proportion of 50 - 99.999 % by weight, preferably 70 - 99.99 % by weight, and the pullulan-cholesterol derivative in a proportion of 0.001 - 50 % by weight, preferably 0.01 - 30 % by weight, with respect to the total amount of the cosmetic product.

Below, the cosmetic products according to the present invention will be described in more detail for ① general description of cosmetics, ② make-up cosmetics, ③ cosmetic products for rouge, ④ cosmetic products for hair conditioning and ⑤ cosmetic products for manicure, in this order. However, the cosmetic products according to the present invention are not restricted to these given below but encompass cosmetics of every form.

### ① General description of cosmetics

The cosmetic products according to the present invention are those in which the above-mentioned pullulan-cholesterol derivative is admixed to commonly known cosmetic products in a known practice. There is no limitation as to the morphologic state of the cosmetic product and quite superior effects may be brought about in any form of cosmetic product. For concrete examples of cosmetics, there may be recited white powdery cosmetics, such as powder foundation, powders for compacts, two-way cake and face powder; local cosmetics, such as eye shadow, powder brush, mascara, lipstick, lipgloss, lip-pencil, eye liner and eyebrow-pencil; emulsified products, such as emulsified foundation and make-up base; partial base cosmetics, such as powder pack, cleansing pack, sun-screen cream, creams, hand cream, swet-suppressing agent and beauty wash lotion; and whole body products, such as baby powder and body powder. Others may be recited, for example, cosmetics for skin care; products for hair treatment, such as hair forming agent, shampoo, rinse, rinse-in-shampoo, hair-shaping lotion, hair spray, hair mousse, hair cream, hair blow and hair oil; and manicures, such as nail enamel.

As the cosmetic products in which the pullulan-cholesterol , derivative is used particularly favorably, there may be exemplified skin care products, such as beauty wash, milky lotion, cream and oil; make-up cosmetics, such as foundation, eye liner, mascara, eyebrow, rouge, cheek rouge and undercoat; and hair care products, such as gel, mousse, spray and hair cream.

In the cosmetic product according to the present invention, cosmetic compounding materials mentioned above to be used in conventional cosmetic products may be incorporated within the range not obstructing the effect of the present invention in accordance with each specific purpose. For the oils, there may be used, for example, hydrocarbon oils, such as squalane, liquid paraffin, vaseline, micro-clistalline wax, ozocerite and ceresine; higher fatty acids, such as myristic acid, palmitic acid, stearic acid, oleic acid, isostearic acis and behenic acid; higher alcohols, such as cetyl alcohol, stearyl alcohol, oleyl alcohol and batyl alcohol; esters, such as cetyl-2-ethyl hexanoate, 2-ethylhexyl palmitate, 2-octyldodecyl myristate, neopentylglycol-2-ethyl hexanoate, trioctanoic acid glyceride, 2-octyldodecyl oleate, isopropyl myristate, myristyl myristate, triisostearic acid glyceride, trioleic acid glyceride, tripalm oil-fatty acid glyceride and cetyl octanoate; fats and oils, such as olive oil, avocado oil, jojoba oil, sunflower oil, liver oil, safflower oil, camellia oil, shea butter, macadamia nut oil, mink oil, lanolin, lanolin acetate, liquid lanolin, castor oil, coconut oil and cotton seed oil; waxes, such as Japan wax and carnauba wax; silicone oils, such as dimethylpolysiloxane, cyclic dimethylpolysiloxane, methylphenylpolysiloxane, polyether-modified silicones, amino-modified silicones, alkyl-modified silicones and fluorine-modified silicones; fluoro-based oils, such as perfluoropolyethers and perfluorocarbons; trimethyl cinnamate; and high polymers, such as silicone resins, high polymeric silicone rubber and acryl-modified silicone copolymers.

In the cosmetic product according to the present invention, it is particularly favorable to use concurrently a water-repellent resin, such as a fluorine-modified silicone, acrylsilicone or a silicone resin, since the polysaccharide-sterol derivative can build up a stout coating film with such water-repellent resin.

It is permissible to incorporate further, for example, moisture-preserving substances, such as ethylene glycol, diethylene glycol, 1,3-butylene glycol, glicerine, hexamethylene glycol, isobutylene glycol, isoprene glycol, polyethylene glycol, propylene glycol, dipropylene glycol, diglycerin, polyglycerin, hyaluronic acid, chondroitin sulfuric acid, pyrrolidone carboxylic acid salt, chitin, urea and chitosan; UV absorbers; and beauty-whitening agent.

Further, it is also permissible to incorporate, each in an adequate proportion, for example, antioxidant, such as tocopherol, butylhydroxyanisole or dibutylhydroxytoluene; an antiseptic agent, such as methylparaben, ethylparaben, propylparaben or butylparaben; powdery additives, such as mica, bentonite, kaolin, talc, mica titanium, bismuth oxychloride and silicic acid anhydride; a pearling agent, such as guanine, pearling laminating resin or pearling agent based on mica-titanium; inorganic pigments, such as ultramarine, chromium oxide and cobalt blue; dyestuffs, such as Sudan III, Quinizarin Green SS and Quinoline Yellow SS; a surfactant, such as that based on Span, based on Tween, based on polyalkyl ether, based on polyoxyethylene-polyoxypropylene, based on fatty acid glyceride or based on polyoxyethylene-glycerin fatty acid ester; a thickening agent, such as carboxyvinyl polymer; medicaments, such as anti-inflammatory agent, vitamins and hormones; and perfumes.

It is furthermore permissible to incorporate, for example, polymers for setting, such as polyvinylpyrrolidone, PVP-VA, vinylmethyl ether/maleic anhydride copolymer, vinyl acetate/crotonic acid copolymer, vinylpyrrolidone/N,N-dimethylaminoethyl methacrylate copolymer diethyl sulfate, N-methacroyloxyethyl-N,N-dimethylammonium/N- α -methylcarboxybetaine/alkylmethacrylate copolymer, vinylpyrrolidone/stearyl acrylate/stearoyloxyethyl-N,N-dimethylamine copolymer.

The pullulan-cholesterol derivative to be used according to the present invention is superior in the film-forming abilty due to the presence of the pullulan segment. Moreover, by using the pullulan-cholesterol derivative according to the present invention in combination with a volatile oil, a coating film can be formed, which reveals no adherent touch or feel after being dried and will not show any tendency to cause transcription onto materials it is brought into contact with. As for the volatile oil, known volatile oils may be used without limitation, for example, light isoparaffin, decamethylpentacyclosiloxane, hexamethyltricyclosiloxane, dimethylpolysiloxane, methylphenylpolysiloxane and organic perfluorocompounds. When the pullulan-cholesterol derivative is compounded in a skin-care or a make-up product, a large effect for improving the preservativity of the dressed state therewith, since a coating film exhibiting higher water-resistant and oil-resistant properties as compared with a coating film formed by a known cosmetic product containing trimethylsiloxy silicic acid can be formed, so that it can be used for all cosmetic products as a cosmetic raw material having ability of general use.

Thus, the pullulan-cholesterol derivative can be used for any form of cosmetic products employed conventionally. As favorable cosmetic products, there may be enumerated, for example, those in which the pullulan-cholesterol derivative is dissolved in a low-boiling cyclic silicone or in a hydrocarbon based on low-boiling isoparaffin, both used as solvent for cosmetics; skin-care cosmetics in which the pullulan-cholesterol, derivative is incorporated in a form of emulsion using a water-soluble polyhydric alcohol or using an emulsifying agent of non-ionic, anionic or cationic nature; cosmetics for rouge made of a composition in which the pullulan-cholesterol derivative is incorporated by being compounded with a solvent for cosmetics, such as a low viscosity silicone; cosmetics for mascara in which the pullulan-cholesterol derivative is incorporated by being compounded in a composition containing, for example, gum arabic, hydroxycellulose, bees wax and black iron oxide; and cosmetics for hair conditioning.

The cosmetic products using the pullulan-cholesterol derivative according to the present invention can be formulated into an emulsified composition of a type of either oil-in-water or water-in-oil by compounding the pullulan-cholesterol derivative, oily components, water-soluble components, purified water and a surfactant and applying techniques of emulsification, solubilization and dispersion within the range not obstructing the purpose of the present invention.

There is no restriction as to the form of the cosmetic products according to the present invention and any voluntary form is permitted, for example, liquid, cream, gel, solid, powder, stick, spray, mousse, aerosol and roll-on type.

The pullulan-cholesterol derivative may be incorporated in the cosmetic product according to the present invention in any voluntary proportion so long as the purpose of the present invention is not obstructed, while it is favorable to incorporate it in a proportion in the range from 0.001 to 50 %, preferably from 0.01 to 30 %, based on the total weight of the cosmetic product, since the moisture-retaining ability due to the hygroscopic and moisture-preserving functions will be high and the lamella-formaing and stabilizing effects are superior at such a proportion together with an effect of suppressing an oily touch or feel on the skin to a lower degree due to the superior film-forming ability.

### ② Make-up cosmetics

The cosmetic product according to the present invention is superior especially for using as a make-up cosmetic. As the make-up cosmetic, those of various forms which are constituted of powdery ingredients and oily ingredients may be recited, wherein there is no special limitation as to the product form, the compounding proportion and so on. There may be recited, for example, solid foundation, powder type foundation, oily foundation, sun-screen foundation, dressing base coat, face powder, cheek rouge, mascara, eye shadow and lip rouge. Emulsified foundations based on emulsifying systems may also be recited. Characteristic feature of the make-up cosmetics resides in a higher content of inorganic powder, such as talc, kaolin, iron oxide, titanium oxide and pearling pigment based on titanium-mica; and organic pigment, such as polyamide (nylon), cellulose and tar-based pigments.

The make-up cosmetics may be obtained conveniently in such a manner that the pullulan-cholesterol derivative is admixed to or incorporated as an alternative component in a cosmetic formulation known conventionally for a make-up cosmetic, in an adequate manner.

For the pullulan-cholesterol derivative to be incorporated in make-up cosmetic product, any one can be used, while those having weight-average molecular weights in the range from 10,000 to 500,000, preferably from 30,000 to 300,000, may be desirable. The content of the pullulan-cholesterol derivative may favorably be in the range from 0.001 to 50 % by weight and most preferably be in the range from 0.01 to 20 % by weight.

The pullulan-cholesterol derivative may be compounded together with a volatile oil. In this way, a make-up cosmetic product is obtained, which is in particular highly extensible and has a crispy feel when applied and which is very excellent in the dressing durability and in the prevention of secondary adhesion. When the amount of cholesteryl group introduced in the pullulan-cholesterol , derivative is short of the proportion of 0.01 group per 100 monosaccharide units constituting the pullulan, satisfactory result with respect to the improvement of dressing durability and to the reduction of secondary adhesion to clothing may not be attained. When the content of the pullulan-cholesterol, derivative is short of 0.001 % by weight, almost no effect for improving the dressing durablity and for reducing the secondary adhesion onto clothing is realized. On the other hand, when the content of the pullulan-cholesterol derivative exceeds over 50 % by weight, the effect for improving the dressing duration and for reducing the secondary adhesion onto clothing may not much increased and will result in reduction in the application performance, such as "extension" of the make-up cosmetic upon the application, so that the fundamental function as a cosmetic is unfavorably obstructed. For preserving the film-forming ability suitably, the content of the pullulan-cholesterol ; should preferably be in the range from 0.001 to 50 % by weight.

There is no special restriction for the volatile oil to be used in the make-up cosmetic product according to the present invention and there may be enumerated, for example, light isoparaffin, decamethylpentacyclosiloxane, octamethyltetracyclosiloxane, hexamethyltricyclosiloxane, dimethylpolysiloxane, methylphenylpolysiloxane and organic perfluorocompounds. Preference is given to volatile hydrocarbon oils having boiling point at normal pressure in the range from 60 to 160 °C.

It is favorable that the content of the volatile oil is in the range from 1 to 90 %, preferably from 5 to 50 %, based on the total weight of the cosmetic product. When the content is short of 1 % by weight, expected effect of increasing the dressing durability and reducing the secondary adhesion is not realized and, on the other hand, when the content is in excess of 90 % by weight, the proportion of the other components necessary for constituting the cosmetic product, such as powdery ingredients, become unfavorably lower and the function as the make-up cosmetic will be lost.

In a make-up cosmetic, oil ingredients, surfactant, colorant, powdery ingredients, waxes, UV absorber, moisture-retaining agent, medicinal ingredients, perfume and stabilizer, which are used conventionally in general for the formulation of make-up cosmetic, may be compounded within the range not obstructing the quality of the cosmetic, such as stability in appearance, viscosity and stiffness.

As the oily ingredients to be used in make-up cosmetic products according to the present invention, there may be recited, for example, liquid oils, solid greases, waxes, hydrocarbon oils, synthetic ester oils and silicone oils. Concrete examples of liquid oils include avocado oil, camellia oil, turtle oil, macadamia nut oil, maize oil, mink oil, olive oil, rapeseed oil, yolk oil, sesame oil, persic oil, wheat germ oil, sazanqua oil, castor oil, linseed oil, sufflower oil, cotton seed oil, perilla oil, soybean oil, peanut oil, tea tree seed oil, kaya oil, rice bran oil, China wood oil, tung oil, jojoba oil, germ oil, triglycerol, trioctanoic acid glyceride and triisopalmitic acid glyceride. As solid greases, there may be recited, for example, cacao butter, coconut butter, horse grease, hardened coconut oil, palm oil, tallow, sheeps grease, hardened tallow, palm kernel oil, lard, bovine bone oil, sumac nut oil, hardened oils, bovine leg oil, sumac wax and hardened castor oil. As waxes, there may be recited, for example, beeswax, candelilla wax, cotton wax, carnauba wax, bayberry wax, insect wax, spermaceti, montan wax, rice-bran wax, lanolin, kapok oil, lanolin acetate, liquid lanolin, sugar cane wax, lanolin fatty acid isopropyl, hexyl laurate, reduced lanolin, jojoba wax, hard lanolin, shellac wax, polyoxyethylene-lanolin alcohol ether, polyoxyethylene-lanolin alcohol acetate, polyoxyethylene-cholesterol ether, lanolin fatty acid polyethylene glycol and polyoxyethylene hydrogenated lanolin alcohol ether. As the hydrocarbon oils, there may be recited, for example, liquid paraffin, ozokerite, squalene, pristane, paraffin, ceresine, squalene, vaseline and microcrystalline wax. As synthetic ester oils, there may be recited, for example, isopropyl myristate, cetyl octanoate, octyl dodecyl myristate, isopropyl palmitate, butyl stearate, hexyl laurate, myristyl myristate, decyl oleate, hexyl decyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid esters, N-alkylglycol monoisostearate, neopentylglycol dicaprate, diisostearyl malate, glycerol di-2-heptylundecanoate, trimethylolpropane tri-2-ethylhexylate, trimethylolpropane triisostearate, pentaneerythritol tetra-2-ethylhexylate, glycerol tri-2-ethylhexylate, trimethylolpropane triisostearate, cetyl 2-ethylhexanoate, 2-ethylhexyl palmitate, glycerol tri-myristate, tri-2-heptylundecanoic acid glyceride, castor oil fatty acid methyl ester, oleic acid oil, cetostearyl alcohol, acetoglyceride, 2-heptylundecyl palmitate, diisobutyl adipate, N-lauroyl-L-glutamic acid 2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate and triethyl citrate. As silicone oils, there may be recited, for example, linear polysiloxanes, such as dimethyl polysiloxane, methyl phenyl polysiloxane and methyl hydrogen polysiloxane; cyclic polysiloxanes, such as decamethyl polysiloxane, dodecamethyl polysiloxane and tetramethyl tetrahydrogen polysiloxane; silicone resins having three dimensional reticular structure; and silicone rubbers.

There is no special limitation for the surfactant to be employed in make-up cosmetic products according to the present invention and, for example, oleophilic non-ionic surfactants and hydrophilic non-ionic surfactants can be employed. As oleophilic non-ionic surfactants, there may be recited, for example, sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, diglycerol sorbitan tetra-2-ethylhexylate; glycerol fatty acid esters, such as glycerol mono (cottonseed oil fatty acid) ester, glycerol monoerucate, glycerol sesquioleate, glycerol monostearate, glycerol α , α*'-*oleic acid pyroglutamic acid ester, glycerol monostearate and glycerol malate; propylene glycol fatty acid esters, such as propylene glycol monostearate and the like; and others including hardened castor oil derivatives, glycerol alkyl ethers and so on.

As the hydrophilic non-ionic surfactants, there may be enumerated, for example, polyoxyethylene sorbitan fatty acid esters, such as polyoxyethylenesorbitan monooleate, polyoxyethylenesorbitan monostearate, polyoxyethylenesorbitan monooleate and polyoxyethylenesorbitan tetraoleate; polyoxyethylenesorbitol fatty acid esters, such as polyoxyethylenesorbitol monolaurate, polyoxyethylenesorbitol monooleate, polyoxyethylene sorbitol pentaoleate and polyoxyethylene monosteatate; polyoxyethylene glycerol fatty acid esters, such as polyoxyethylene glycerol monostearate, polyoxyethylene glycerol monoisostearate and polyoxyethylene glycerol triisostearate; polyoxyethylene fatty acid esters, such as polyoxyethylene monooletate, polyoxyethylene distearate, polyoxyethylene monodioleate and ethylene glycol cis-stearate; polyoxyethylene alkyl ethers, such as polyoxyethylene lauryl ether, polyoxyethylene oleyl ether, polyoxyethylene stearyl ether, polyoxyethylene behenyl ether, polyoxyethylene 2-octyldodecyl ether and polyoxyethylene cholestanol ether; polyoxyethylene alkyl phenyl ethers, such as polyoxyethylene octyl phenyl ether, polyoxyethylene nonyl phenyl ether and polyoxyethylenedinonyl phenyl ether; Pluronic types, such as Pluronics; polyoxyethylene-polyoxypropylene alkyl ethers, such as polyoxyethylene-polyoxypropylene cetyl ether, polyoxyethylene-polyoxypropylene 2-decyltetradecyl ether, polyoxyethylene-polyoxypropylene monobutyl ether, polyoxyethylene-polyoxypropylene hydrogenated lanolin and polyoxyethylene-polyoxypropylene glycerol ether; tetrapolyoxyethylene-tetrapolyoxypropylene ethylenediamine condensation products, such as Tetronics; polyoxyethylene-hydrogenated castor oil derivatives, such as polyoxyethylene castor oil, polyoxyethylene-hydrogenated castor oil, polyoxyethylene-hydrogenated castor oil monoisostearate, polyoxyethylene-hydrogenated castor oil triisostearate, polyoxyethylene-hydrogenated castor oil monopyroglutamic acid monoisostearic acid diester and polyoxyethylene-hydrogenated castor oil maleate; polyoxyethylenebeeswax-lanolin derivatives, such as polyoxyethylenesorbitol beeswax and the like; alkanolamides, such as palm oil fatty acid diethanolamide, lauric acid monoethanolamide and fatty acid isopropanolamides; and others including polyoxyethylene-propylene glycol fatty acid esters, polyoxyethylene alkylamines, polyoxyethylene fatty acid amides, sucrose fatty acid esters, polyoxyethylene-nonylphenyl formaldehyde condensation products, alkylethoxydimethylamine oxides and trioleyl phosphate.

As the powdery ingredients to be incorporated in make-up cosmetic products according to the present invention, known ones employed usually for make-up cosmetics can be used without limitation. Concrete examples include inorganic powdery substances, such as talc, kaolin, mica, sericite, muscovite, phlogopite, lepidlite, biotite, lithia mica, vermiculite, magnesium carbonate, calcium carbonate, aluminum silicate, magnesium metasilicate aluminate, barium silicate, calcium silicate, magnesium silicate, strontium silicate, metal salts of wolframate, magnesium, silica, zeolites, barium sulfate, calcined calcium sulfate (calcined gypsum), calcium phosphate, fluorapatite, hydroxyapatite, ceramic powder, metal soaps (zinc myristate, calcium palmitate, aluminum stearate and so on) and boron nitride; oranic powdery materials, such as polyamide powder (powdery nylons), polyethylene powder, polymethylmethacrylate powder, polystyrene powder, powdery product of copolymer resin of styrene and acrylic acid, powder of benzoguanamine resin, polytetrafluoroethylene powder and cellulose powder; inorganic white pigments, such as titanium dioxide and zinc oxide; inorganic red pigments, such as iron oxide (red iron oxide), iron titanate; inorganic brown pigments, such as γ-iron oxide etc.; inorganic yellow pigments, such as yellow iron oxide and loess; inorganic black pigments, such as black iron oxide, carbon black and titanium lower oxides; inorganic purple pigments, such as mango violet and cobalt violet; inorganic green pigments, such as chromium oxide, chromium hydroxide and cobalt titanate; inorganic blue pigments, such as ultramarine and prussian blue; pearling pigments, such as titanium oxide-coated mica, titanium oxide-coated bismuth oxychloride, titanium oxide-coated talc, colored titanium oxide-coated mica, bismuth oxychloride and scale flake; powder metal pigments, such as aluminum powder and copper poweder; organic pigments, such as Red 201, Red 202, Red 204, Red 205, Red 220, Red 226, Red 228, Red 405, Orange 203, Orange 204, Yellow 205, Yellow 401 and Blue 404; organic pigments, for example, zirconium-, barium- and aluminum-lakes, such as Red 3, Red 104, Red 106, Red 227, Red 230, Red 401, Red 505, Orange 205, Yellow 4, Yellow 5, Yellow 202, Yellow 203, Green 3 and Blue 1; and natural pigments, such as chlorophyll and β-carotin. Also powders may be employed, in which the surfaces of the powder particles are subjected to a hydrophobicating treatment in a usual manner by, for example, silicone resin treatment, wax treatment, dextrin-fatty acid treatment and fluorine treatment.

As waxes to be incorporated in make-up cosmetic products according to the present invention, known waxes used in make-up cosmetics can be used without any limitation. Concrete examples include beeswax, candelilla wax, cotton wax, carnauba wax, bayberry wax, insect wax, spermaceti, montan wax, rice-bran wax, lanolin, kapok wax, lanolin acetate, liquid lanolin, sugarcane wax, lanolin fatty acid isopropyl, hexyl laurate, reduced lanolin, jojoba wax, hard lanolin, shellac wax, polyoxyethylene lanolin alcohol ether, polyoxyethylene lanolin alcohol acetate, polyoxyethylene cholesterol ether, lanolin fatty acid polyethylene glycol and polyoxyethylene hydrogenated lanolin alcohol ether, ceresine, vaseline and microcrystalline wax. In general, the content of wax may favorably be 1 - 30 %, based on the total weight of the make-up cosmetic product.

As the UV absorber to be incorporated in make-up cosmetic products according to the present invention, known UV absorbers used for make-up cosmetics may be employed without any limitation. Concrete examples include UV absorbers based on benzoic acid, such as paraaminobenzoic acid (in the following, abbreviated as PABA), PABA monoglycerol ester, N,N-dipropoxy-PABA ethyl ester, N,N-diethoxy-PABA ethyl ester, N,N-dimethyl-PABA butyl ester, N,N-dimethyl-PABA methyl ester; UV absorbers, based on anthranilate, such as homomenthyl-N-acetyl anthranilate and so on; UV absorbers based on salicylate, such as amyl salicylate, menthyl salicylate, homomenthyl salicylate, octyl salicylate, phenyl salicylate, benzyl salicylate and p-isopropanolphenyl salicylate; UV absorbers based on cinnamate, such as octylmethoxy cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl- α-cyano- β -phenyl cinnamate, 2-ethylhexyl- α-cyano- β-phenyl cinnamate and glyceryl-mono-2-ethylhexanoyl-diparamethoxy cinnamate; UV absorbers based on benzophenone, such as 2,4-dihydroxybenzophenone, 2,2'-dihydroxy-4-methoxybonzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sufonic acid salts, 4-phenylbenzophenone, 2-ethylhexyl-4'-phenylbenzophenone-2-caboxylate, 2-hydroxy-4-n-octoxybenzophenone and 4-hydroxy-3-carboxybenzophenone; and 3-(4'-methylbenzylidene)-d,1-camphor, 3-benzylidene-d,1-camphor, urocanic acid, urocanic acid ethyl ester, 2-phenyl-5-methylbenzoxazole, 2,2'-hydroxy-5-methylphenylbenzotriazole, 2-(2'-hydroxy-5'-tert-octylphenyl)benzotriazole, 2-(2'-hydroxy-5'-methylphenyl)benzotriazole, dibenzal azine, dianisoylmethane, 4-methoxy-4'-tert-butyldibenzoylmethane and 5-(3,3-dimethyl-2-norbornylidene)-3-pentane-2-one.

As the moisture-preserving ingredients to be incorporated in make-up cosmetic products according to the present invention, known ones used in make-up cosmetics can be employed without any limitation. Concrete examples include polyethylene glycol, propylene glycol, glycerin, 1,3-butylene glycol, xylitol, sorbitol, maltitol, chondroitin sulfuric acid, hyaluronic acid, mucoitin sulfuric acid, caronic acid, atherocollagen, cholesteryl-12-hydroxystearate, sodium lactate, salts of bile acid, dl-pyrrolidone carboxylic acid salts, soluble short-chain collagen, diglycerol-(ethylene oxide)-propylene oxide adduct, Rosa roxburghii glabra extract, extract of Achillea millefolium and melilote extract.

As the medicinal ingredients to be incorporated in make-up cosmetic products according to the present invention, known ones used for make-up cosmetics can be employed without any limitation. Concrete examples include blood circulation promotors, such as nonylic acid valerylamide, nicotic acid benzyl ester, nicotic acid β -butoxyethyl ester, capsaicine, zingerone, cantharis tincture, ichthammol, caffeine, tannic acid, α-borneol, tocopherol nicotinate, inositol hexanicotinate, cyclandelate, cinnarizine, tolazoline, acetylcholine, verapamil, cepharanthin and γ-oryzanol; antimycotic agents, such as clotrimazole, pentachlorophenol, trichlorophenol caproate, tribromophenol caproate, lauryltriphenylphosphonium bromide, diantazole hydrochloride, p-acethylaminophenyl rhodan, thimerosal, undecylenic acid, zinc undecylenate, dermacid, valithion, pullolnitron, siccanin, miconazole, econazole, isoconazole, sulconazole, thioconazole, bifonazole, oxyconazole, ketoconazole, ciclopiroxolamine, torciclate, naphtifin, griseofulvin and 5-fluorocytosine; and whiting beauty agent, such as arubtin, kojic acid, placenta extract and vitamin C and derivative thereof.

The pullulan-cholesterol derivative to be employed according to the present invention exhibits an ultraviolet rav absorbing function in the wave length range originated from that of the constituent cholesterol and reveals, when solubilized in a specific medium to be incorporated as a compoment of the cosmetic product, a water-repellent property, a lubricity and a gloss-imparting ability. It is very useful as a coating film forming ingredient for a raw material of the cosmetic product, since it has at the same time a better coating film-forming ability due to the pullulan segment, so that this characteristic feature may be made use of, when pertinent form of make-up cosmetic is chosen.

### ③ Cosmetic products for lip rouge

The cosmetic product according to the present invention is especially superior also for use as lip rouge cosmetic. The lip rouge cosmetic product according to the present invention can be incorporated in any type of lip rouge or as a raw material in any type of lip rouge. The cosmetic product for lip rouge according to the present invention is constituted of, in addition to the pullulan-cholesterol derivative, cosmetic ingredients employed in general, such as waxes, liquid oils, pigments, pearling agent and so on. They can be processed into a lipstick by shaping in a form of rod or, alternatively, can be formed into a lip rouge in a form of a dish permitting precise drawing of lip contour using a drawing tool by being formed within a metal pan. While such lip rouge cosmetics are made predominantly of oily ingredients, they may contain further a volatile solvent, such as a volatile silicone or volatile hydrocarbon; and a coating film-forming ingredient, such as a wax or an organic silicone resin. It is futhermore possible to formulate the lip rouge cosmetic in a form of an emulsion lip rouge by blending water therewith or in a form of a liquid lip rouge by blending a specific oily ingredient and a water-soluble substance with a water-in-oil type rouge. The cosmetic product for lip rouge may favorably be formulated also in a form of a paste or a liquid of a type of water-in-oil. By admixing the pullulan-cholesterol derivative adequately to any type of lip rouge known hitherto or by formulating it as an alternative component therewith, it is able to obtain a cosmetic product for lip rouge, in which the dressed film is strengthened, adherent touch feel is excluded, color-transference phenomenon is improved and the dressed film is lustrous so that the cosmetic function as a lip rouge is improved so as to impart a wet feel to the lip. The cosmetic product for lip rouge according to the present invention can be used in any composition or in any form, so long as the pullulan-cholesterol derivative is contained. As one of favorable example, a water-in-oil type cosmetic product for lip rouge of a form of liquid or paste is recited, in which a volatile silicone oil, a pullulan-cholesterol derivative and an organo-modified clay mineral are incorporated in an oily component and, at the same time, a surfactant based on polyoxyalkylene-modified organopolysiloxane as a dispersant for powder is incorporated. Such a water-in-oil type cosmetic product for lip rouge can be used so as to permit to draw smoothly the lip contour precisely when a drawing tool is used. It is better in the application performance and in the placticability with better stability and will result in a lustrous coating film exhibiting no color transference and revealing durable dressing without deteriorating wetness of the lip.

The cosmetic materials for lip rouge can be formulated into any cosmetic product for lip rouge of any voluntary composition so long as it contains the pullulan-cholesterol derivative, wherein a favorable example of the composition of lip rouge cosmetic product comprises 5 - 60 % by weight of a volatile silicone oil, 0.001 .- 50 % by weight of a pullulan-cholesterol derivative, 0.1 - 15 % by weight of a surfactant based on polyoxyalkylene-modified organopolysiloxane, 0.1 - 7 % by weight of an organo-modified clay mineral, 2 - 60 % by weight of water and 2 - 40 % by weight of a powdery ingredient for cosmetic use.

As the volatile silicone oil, there may favorably be recited among cyclic and linear dimethylpolysiloxanes those exhibiting high volatilizing velocity at normal temperature. As the cyclic dimethylpolysiloxane, there may be recited, for example, octamethylcyclotetrasiloxane [abbreviated hereinafter to cyclic silicone (tetramer)] and decamethylcyclopentasiloxane [abbreviated in the following to cyclic silicone (pentamer)]. As the linear dimethylpolysiloxane, there may be exemplified those having a viscosity at 25 °C of 5 mm²/sec or less. For the volatile silicone oil, it is permissible to use linear one and cyclic one in combination. It is favorable that the content of the volatile silicone oil is in the range from 5 to 60 % by weight, preferably from 10 to 50 % by weight. When the content is less than 5 % by weight, the cosmetic product for lip rouge will have higher viscosity and may sometimes become difficult to realize application using a drawing tool, resulting in a deterioration in the application performance, though may occasionally be usable without problem. When the content is greater than 60 % by weight, there may sometimes occur unfavourable irritation by the volatile silicone oil on skin, though occasional use thereof may be permitted without problem.

When cyclic silicone oil is used, it is favorable to choose the proportion of the cyclic silicone (tetramer) relative to the cyclic silicone (pentamer) in the range from 8/2 to 2/8, though not restricted thereto only. When the proportion is in the above range, the product exhibits better stability at low temperatures with proper volatilizing velocity and may result in occasionally better application aspect.

For the pullulan-cholesterol derivative to be incorporated in the cosmetic product for lip rouge according to the present invention, any one can be employed, but those which have number average molecular weights in the range from 10,000 to 1,000,000, preferably from 30,000 to 300,000, may be favorable. The content of the :pullulan-cholesterol derivative may favorably be in the range from 0.001 to 50 % by weight, preferably from 0.01 to 20 % by weight. When the content is lower than 0.001 % by weight, the coating film strength is lower and the durability of the dressing becomes deteriorated. When the content is greater than 50 % by weight, the cosmetic product for lip rouge may sometimes have too high a viscosity and deteriorate the application performance using a drawing tool. For maintaining the moisture-preservativity and the coating film-forming ability each at an adequate level, the content of the pullulan-cholesterol derivative may favorably be in the range from 0.001 to 50 % by weight.

The surfactant based on the polyoxyalkylene-modified organopolysiloxane (referred to hereinafter as POA-modified silicone) may sometimes be denoted as polyether-modified silicone or alkylpolyether-modified silicone and functions as an emulsifier or a dispersant. As the POA-modified silicone, there may favorably be used those which are in a form of liquid or paste, in particular, those which are insoluble in water, for example, SILICONE KF-945 A (trademark, a product of Shin-Etsu Chemical Co., Ltd.), SILICONE SH-3772 C and SILICONE SH-3775 C (both trademarks, products of Toray-Dow Corning Silicone K.K.) and ABIL WE-09 (trademark, a product of the firm Goldschmidt). These POA-modified silicones have each a main skeleton chain of polysiloxane and exhibit better compatibility with the volatile silicone and with the polysaccharide-sterol derivative present in the oil phase, so that a better powder dispersing stability may occasionally be expectable. There is no special limitation in the content of the POA-modified silicone, while a content in the range from 0.1 to 15 % by weight, preferably from 0.5 to 10 % by weight, may be favorable. When the content is less than 0.1 % by weight, the function as a dispersant may become insufficient and may occasionally result in deterioration of stability. When the content is greater than 15 % by weight, the dressed coating film may sometimes suffer from collapse by sweat or so on and may result in deterioration of durability.

For the organo-modified clay mineral, any one which is used usually in cosmetic products can be incorporated. There may be exemplified those in which natural or synthetic clay minerals, such as montmorillonite, saponite, hectorite and bentonite, are modified by exchanging the ion-exchangeable cation therein with an organic polar compound or an organic cation. They may occasionally provide the oil phase, namely, the outer phase, with some structure which contributes to an increase in the stability of the cosmetic product. While there is no special limitation in the content thereof, a content in the range from 0.1 to 7 % by weight, preferably from 0.5 to 5 % by weight may be favorable. When the content is less than 0.1 % by weight, formation of the structure may occasionally not be attained and the stability may be deteriorated. When the content is greater than 7 % by weight, the cosmetic product for lip rouge may occasionally become stiffer, with the result of deterioration of the application performance using drawing tool.

While there is no special limitation in the content of water in the cosmetic product of lip rouge, a water content in the range from 2 to 60 % by weight, preferably from 4 to 50 % by weight may be favorable. When the content is less than 2 % by weight, it becomes not able to impart a wet feel to the lip and the lip becomes dry. When the content is greater than 60 % by weight, the proportion of the inner phase becomes too high, resulting in deterioration of the stability. It is permisible that the aqueous system of the inner phase contains, in addition to water, water-soluble ingredients, such as alcohols, polyhydric alcohols, acids, their salts, alkalis, water-soluble high molecular weight substances, coloring matters, moisture-preserving agent, antiseptics and water-soluble medicaments, under voluntary choice, each within a content not obstructing the effect of the present invention.

For the powdery ingredients for the cosmetic products, those which are used usually in cosmetics can be employed without any limitation. There may be compounded, for example, those which function not only as coloring materials for lip rouge but also as the agent for regulating drying velocity. Concrete examples of the powdery ingredients include extender pigments, such as talc, sericite, kaolin, mica, magnesium carbonate, calcium carbonate, magnesium silicate, magnesium alminosilicate, silica and synthetic mica; white pigments, such as titanium oxide, zinc oxide and barium sulfate; inorganic coloring pigments, such as red iron oxide, yellow iron oxide, black iron oxide, ultramarine and prussian blue; organic coloring pigments, such as tar pigment and so on; pearling pigments, such as mica titanium, iron oxide mica titanium and bismuth oxychloride; and organic powdery substances, such as nylon powder, silk powder, styrene powder and crystalline cellulose. In using in the cosmetic product according to the present invention, the inorganic powdery ingredients among those given above may occasionally be more favorable when they are subjected to a hydrophobicating surface treaement using an oil, a silicone or a fluoro-based compound, since thereby the dispersion of the inorganic powdery ingredient in the oil phase may be improved. When the blending proportion of powdery ingredient of lower hydrophobicity is higher, the stability of the cosmetic products for lip rouge may occasionally be decreased. While there is no special limitation in the content of the powdery ingredient for cosmetics, a content in the range from 2 to 40 % by weight, preferably from 5 to 30 % by weight, may be favorable. When the content is less than 2 % by weight, the drying velocity of the lip rouge may occasionally be decreased and the color development may also be reduced to thereby render it unadapted for use as lip rouge. When the content is greater than 40 % by weight, the viscosity will become too high to be spooned by a drawing tool and the dressed coating film may become nonuniform to render the beautiful appearance or the durability of the dressing deteriorated, though the drying velocity is increased.

It is possible to incorporate in the cosmetic products for lip rouge according to the present invention ingredients which are liquid or semisolid at normal temperature as non-volatile ingredients other than the ingredients given above. For such non-volatile ingredients, oils and water-soluble substances used usually in cosmetic products may be recited, though not restricted thereto. Concrete examples of oils include liquid oils and semisolid oils of those based on hydrocarbon, based on ester, based on triglyceride and based on silicone, such as liquid paraffin, squalane, vaseline, polybutene, glyceryl trioctanoate, propylene glycol dicaprate, cetyl 2-ethylhexanoate, isocetyl stearate, dipentaerythritol fatty acid esters, jojoba oil, dimethylpolysiloxane and methylphenylpolysiloxane. As the water-soluble substances, there may be recited, for example, polyhydric alcohols, polyethylene glycol, polyglycerols and esters of them. It is possible to incorporate waxes, resins, tallows, fatty acids and higher alcohols, so long as the resulting composition mixed with the non-volatile ingredient is present in a form of liquid or semisolid at normal temperature. In case non-volatile ingredients are incorporated, the content thereof is not specifically restricted, while a preferred content may be in the range from 3 to 30 % by weight. By incorporating these ingredients, the luster and also the sealability of the dressed coating film may occasionally be improved, whereby the effect of maintaining the wet state of the lip can be increased.

It is possible to incorporate in the cosmetic products for lip rouge according to the present invention ingredients which can be incorporated usually in cosmetic products, so long as the effect of the cosmetic product is not obstructed. There may be recited, for example, natural coloring materials, ultraviolet ray absorber, moisture-preserving agent, cool-feeling agent, antiseptic, antioxidant, surfactant, perfumes, medicinal ingredients, such as vitamins and hormones, oil-gelating agent and pH-regulator.

The morphological state of the cosmetic product for lip rouge according to the present invention is not specifically limited and any form is possible so long as it belongs to ones known hitherto. The form of the cosmetic product for lip rouge can voluntarily be chosen under provision of creative possibilities for the kind and content of each component to be compounded without any respect to those given above for the lip rouge cosmetics, so long as the pullulan-cholesterol derivative is contained.

### ④ Cosmetic products for hair conditioning

The cosmetic materials according to the present invention are in particular superior also for use as cosmetic products for hair conditioning. The pullulan-cholesterol derivative is constituted mainly of the pullulan and contains a large proportion of the cholesteryl group, and, thus, has properties similar to a substance, such as an amino-modified silicone or the like, so that it provides better adsorption onto hair and is superior in the adhesion and in the resistance to washing together with superior effects of improving smooth combing and water-retentivity and of suppressing occurrence of electrostatic charge on combing.

In case the cosmetic materials according to the present invention are used as a cosmetic product for hair conditioning, the contemplated cosmetic product for hair conditioning having a content of the pullulan-cholesterol derivative can be prepared by adding the pullulan-cholesterol derivative adaptively to, or incorporating it as an alternative component, in a formulation of components known usually for a cosmetic product for hair conditioning.

The pullulan-cholesterol derivative may be present in a soft consistency, such as that exhibited by a dimethylsiloxane of a high molecular weight of, for example, 400,000 - 700,000, and can form a soft and non-adherent coating film, even when the pullulan-cholesterol derivative has a high molecular weight of 20,000 to 50,000 or so, by controlling the content of the cholesteryl group in the pullulan-cholesterol derivative. When the pullulan-cholesterol derivative is compounded in, for example, a coating agent for branched hair or a rinse-in-shampoo, such effects as repairing the branching portion of hair by binding together, improving smooth combing, preventing desquamation of cuticles from hair on brushing and preventing formation of branched hair and a hair lustering effect may be attained. These effects may assumably be revealed by formation of a coating film of the pullulan-cholesterol derivative on the surface of hair.

The cosmetic products for hair conditioning according to the present invention may be present in any form without specifically respecting the form, so long as the form is known hitherto. The cosmetic products for hair conditioning according to the present invention may be made of a composition of various application types. They may be in forms of, for example, general hair conditioning products, shampoo products, rinse products, treatment products, setting products, permanent-waving liquid and mascara. They may have forms of liquid, cream, aqueous emulsion and gel.

The cosmetic products for hair conditioning according to the present invention may be composed solely of the pullulan-cholesterol derivative as polymeric component. It is also possible to use the pullulan-cholesterol derivative concurrently with a natural or synthetic polymeric component or a modified natural polymeric substance for hair conditioning known heretofore. It is further possible to use concurrently therewith a plasticizer, such as a higher fatty acid ester, glycerin or a polyethylene glycol. Further, it is also possible to use various additives concurrently, for example, surfactants, thickening substances, hydrotropes, emulsions, conditioners, oil and fatts, moisture-preserving agents, coloring agents, antiseptics and perfumes.

If the cosmetic products for hair conditioning are shampoo, rinse, treatment agent, setting agent, permanent-waving agent and mascara, they may be prepared by adding the pullulan-cholesterol : derivative to known compositions therefor in an amount of 0.001 % by weight or more, to use them as, for example, shampoo or mascara, containing the pullulan-cholesterol derivative.

Particularly favorable application forms of the cosmetic products for hair conditioning according to the present invention include those of hair spray of aerosol type, hair spray of pump type, foaming aerosol, hair mist, set lotion, hair-styling gel, hair liquid, hair cream and hair oil, wherein they may be present in any voluntary form including those based on solubilization, based on emulsification, based on powder dispersion, based on two phases of oil/water and based on three phases of oil/water/powder. In the case of that based on emulsification, an oil phase containing the pullulan-cholesterol derivative is emulsified using an emulsifier of surfactant of, for example, non-ionic, cationic or anionic nature or of mixture thereof, to form a product based on emulsification. On the emulsification, it is also possible to form the emulsion in such a way that an emulsifier is dissolved in a water-soluble polyhydric alcohol, whereto an oil phase containing the pullulan-cholesterol derivative is added and the resulting mixture is emulsified to form an emulsified composition, whereupon the resulting composition is diluted with an aqueous phase containing a cationic resin. While it is possible to use the pullulan-cholesterol; derivative as a surface active agent, it is quite indifferent to use a known emulsifier used usually.

As the emulsifier used for emulsification other than the pullulan-cholesterol derivative, any one may be used so long as it is known, for which there may be recited, for example, nonionic surfactants, such as polyoxyethylene alkyl ethers, polyoxyethylene alkyl phenyl ether, polyoxyethylene cholesteryl ether, polyoxyethylene sorbitan fatty acid esters, polyoxyethylene glyceryl fatty acid esters, polyoxyethylene hardened castor oil, polyethylene glycol fatty acid esters, polyglycerol fatty acid esters, sucrose fatty acid esters and polyether-modified silicones; cationic surfactants, such as stearyltrimethylammonium chloride, distearyldimethylammonium chloride, cetyltrimethylammonium chloride, behenyltrimethylammonium chloride and cetyl pyridinium chloride; and anionic surfactants, such as sodium cetyl sulfate, sodium polyoxyethylene lauryl ether sulfate, sodium lauryl sulfate, potassium salt of coconut oil fatty acid and sodium coconut oil fatty acid methyltaurate.

As the water-soluble polyhydric alcohols, there may be enumerated, for example, ethylene glycol, propylene glycol, 1,3-butylene glycol, 1,4-butylene glycol, dipropylene glycol, glycerol, diglycerol, triglycerol, tetraglycerol, glucose, maltose, maltitol, sucrose, fructose, xylitol, sorbitol, maltotriose, threitol, erythritol, starch, decomposed sugar reduced alcohol and hyaluronic acid. They can be used either alone or in combination of two or more of them.

As the pullulan-cholesterol derivative to be incorporated in the cosmetic products for hair conditioning, those having a weight-average molecular weight in the range from 10,000 to 1,000,000, preferably from 10,000 to 500,000, may be favorable. The amount of cholesteryl groups introduced in the pullulan-cholesterol derivative may favorably be in the range from 0.05 to 15 groups, more preferably from 0.1 to 10 groups, per 100 monosaccharide units constituting the pullulan. When the introduced proportion of the Cholesteryl groups is short of 0.01 groups, the interaction with hair may in some cases become insufficient and the preservativity of the effect for preventing hair damage is deteriorated.

The content of the pullulan-cholesterol derivative in the entire mass of the cosmetic product for hair conditioning may favorably be in the range from 0.001 to 50 % by weight, preferably from 0.01 to 30 % by weight. When the content is short of 0.001 % by weight, the contemplated effect will not be attained sufficiently. When the content exceeds over 50 % by weight, it becomes difficultly soluble. For use as an agent for rinsing effect, it may favorably be in the range from 0.001 to 50 %, preferably from 0.01 to 30 %, based on the total weight of the cosmetic product for hair conditioning. When the content is short of 0.001 % by weight, sufficient effect may not be obtained and, when it exceeds over 50 % by weight, dissolution of the pullulan-cholesterol derivative becomes unfavorably disturbed. The content of the pullulan-cholesterol derivative may favorably be in the range from 0.001 to 50 % by weight, in order to maintain the water-retentivity and the coating film-forming ability adequately.

On blending the pullulan-cholesterol derivative with the cosmetic materials for hair conditioning, it may be blended after having been dissolved in an oil of liquid form. It is of course possible to add it to any cosmetic material for hair conditioning isolately, before it is dissolved in the formulation system. As the oil of liquid form, there may be recited, for example, linear silicones, cyclic silicones and hydrocarbons based on isoparaffin. Concrete examples of the linear silicones include octamethylcyclotetrasiloxane, decamethylcyclopentasiloxane and tetradecamethylcyclohexasiloxane.

As the hydrocarbons based on isoparaffin, those having boiling points at normal pressure in the range from 60 to 260 °C may be used. Concrete examples include ISOPAR A (trademark, a product of Exxon corp.), ISOPAR C (do.), ISOPAR D (do.), ISOPAR E (do.), ISOPAR G (do.), ISOPAR H (do.), ISOPAR K (do.), ISOPAR L (do.), and ISOPAR M (do.), SHELLSOL 71 (trademark, a product of Shell Oil Co.) and SORTOL 100, 130 and 220 (trademark, all are products of Phillips Petroleum Co.). The hydrocarbons based on isoparaffin may be used either solely of voluntary one or in a combination of two or more of them, wherein the content of total sum of the hydrocarbons based on isoparaffin may favorably be in the range from 1 to 50 times weight the pullulan-cholesterol derivative and may preferably be in the range from 10 to 80 %, based on the total weight of the cosmetic product for hair conditioning. In case the cosmetic product for hair conditioning is a hair wash, a content of 20 % by weight or less may be favorable.

The cosmetic products for hair conditioning according to the present invention may further contain, within the range in proportional and qualitative sense not obstructing the effects of the present invention and in accordance with each specific purpose, other ingredients in addition to the constituent components given above. For example, there may be recited oily ingredients, such as liquid paraffin, squalane, lanolin derivatives, higher alcohols, various ester oils, avocado oil, palm oil, beef tallow, jojoba oil, silicone oils, polyalkylene glycol polyethers and carboxylic acid oligoesters thereof and hydrocarbons based on terpene; water-soluble polyhydric alcohols, such as ethylene glycol, propylene glycol, 1,3-butylene glycol, glycerol, sorbitol and polyethylene glycol; moisture-preserving agents, such as hyaluronic acid, chondroitin sulfuric acid and pyrrolidone carboxylate; UV absorbers; UV scattering agents; resins, such as resins based on acrylate, silicone resins and polyvinylpyrrolidone; proteins and protein degradation products, such as soybean proteins, gelatine, collagen, silk fibroin and elastin; antiseptics, such as ethylparaben and butylparaben; activating agents, such as various amino acids, biotin and pantothenic acid derivatives; blood flow promotors, such as γ -oryzanol, sodium dextran sulfate, vitamin E derivatives and nicotinic acid derivatives; antiseborrheic agents, such as sulfur and thiantol; diluents, such as ethanol, isopropanol and tetrachlorodifluoroethane; thickning agents, such as carboxyvinyl polymers; others including medicaments, perfumes and colorants.

### ⑤ Cosmetic products for manicure

The cosmetic materials according to the present invention are in particular superior also for use as cosmetic material for manicure. In case the cosmetic materials according to the present invention is used as the cosmetic material for manucure, a cosmetic product for manicure containing the pullulan-cholesterol derivative can be obtained by preparation by admixing the pullulan-cholesterol derivative in a pertinent manner to, or incorporating it, as an alternative component, in a mixture of components known usually for a cosmetic product for manicure.

For the pullulan-cholesterol derivative to be used for the cosmetic product for manicure, any one can be employed whereas those having a weight-average molecular weight in the range from 10,000 to 1,000,000, preferable from 30,000 to 500,000 may favorably be used, since they improve aspects for peeling off, for the smooth appearance and for the luster. The content of the pullulan-cholesterol derivative in the cosmetic product for manicure may favorably be in the range from 0.001 to 50 %, preferably from 0.01 to 30 % by weight, based on the total weight of the cosmetic product. For maintaining the water-retentivity and the coating film-forming ability in an adaptable condition, the content of the pullulan-cholesterol derivative should favorably be in the range from 0.001 to 50 % by weight.

The pullulan-cholesterol derivative to be used for the cosmetic product for manicure may be incorporated after having been dissolved in a solvent system used usually in manicure liquid. Such a solvent system is constituted essentially of a mixture of various volatile organic solvents for reducing the drying time relatively short. While such solvents may adequately be chosen among volatile organic solvents known usually, preference is given to aceton, ethyl acetate, butyl acetate, 2-methoxyethyl acetate, methyl ethyl ketone, methyl isobutyl ketone, methyl acetate, amyl acetate and isopropyl acetate. For the solvent system, that containing a diluent is preferred. As the diluent, saturated linear and branched chain hydrocarbons, such as hexane and octane; and aromatic hydrocarbons, such as toluene and xylene, may be exemplified. The solvent systen may comprise other volatile solvents. There is no special limitation for the volatile solvent, it may comprise, for example, ethanol, n-butanol, n-propanol, isopropanol and mixture of them.

In the cosmetic product for manicure, a coating film-forming material may also be incorporated. While the pullulan-cholesterol, derivative has in itself a coating film-forming ability, it is no matter that another coating film-forming material known usually is further incorporated. As the coating film-forming material, there may be recited, for example, nitrocellulose products, in particular, of "RS" and "SS" types. As the additionally incorporated coating film-forming material, polyvinyl derivatives, such as polyvinyl butyrate etc., may also be employed.

In the cosmetic product for manicure, a plasticizer may also be incorporated. The content of the plasticizer may favorably be in the range from 2 to 10 %, based on the total weight of the cosmetic produt. For the plasticizer, any one may be incorporated, so long as it permit adjustment of the flexibility of the coating film without reducing the physical strength of the coating film. As the plasticizer incorporated favorably in the cosmetic product for manicure according to the present invention, there may be recited, for example, tricresyl phosphate, benzyl benzoate, tributyl phosphate, butyl acetylricinoleate, glyceryl acetylricinoleate, dibutyl phthalate, butyl glycolate, dioctyl phthalate, butyl stearate, tributoxyethyl phosphate, triphenyl phosphate, triethyl citrate, tributyl citrate; tributyl acetylcitrate, tri(2-ethylhexyl) acetylcitrate, dibutyl tartarate, dimethoxyethyl phthalate, diisobutyl phthalate, diamyl phthalate, camphor, glycerol triacetate and mixtures of them.

In the cosmetic product for manicure according to the present invention, such resins as used in general in cosmetic products for manicure may be incorporated. Any resin may be incorporated for the resin to be used, so long as it is suitable, wherein arylsulfonamide-formaldehyde resins and alkyd resins may be exemplified. The content of the resin may favorably be in the range from 0.5 to 15 %, based on the total weight of the cosmetic product for manicure. As the resin of arylsulfonamide-formaldehyde type, well known toluenesulfonamide-formaldehyde resin may be enumerated. Such resins improve the luster and adhesion, while at the same time incresing the coating film-forming performance.

The cosmetic product for manicure according to the present invention may either be transparent or be colored. For coloration, one or more kinds of natural or synthetic pigments known per se may be incorporated. As the pigment, any one may be employed, so long as it is a pigment known usually for cosmetic product. As the organic pigment, for example, D&C Red 5, D&C Red 6, D&C Red 7, D&C Red 10, D&C Red 11, D&C Red 12, D&C Red 13 and D&C Red 34; and lake pigments, such as Lake D&C yellow 5 and Lake D&C Red 2. As other organic pigment, guanine and others may also be recited. As the inorganic pigment, for example, titanium dioxide, bismuth oxychloride, brown iron oxide and red iron oxide may be recited. The content of the pigment is determined according to each specific purpose of the cosmetic product for manicure in an adequate manner, while it may favorably be in the range from 0.01 to 2 %, based on the total weight of the cosmetic product.

In the cosmetic product for manicure according to the present invention may further be incorporated a thixotroping agent in some content in order to avoid sedimentation of pigment. For the thixotroping agent, any known one may be incorporated within the range not obstructing the purpose of the present invention, so long as it is adequate, wherein the content thereof may adequately be chosen. The cosmetic product for manicure according to the present invention may contain further additives used ususally in manicure liquid. For such additive, any known ones chosen in accordance with each specific purpose may be employed and contained, so long as they are those contained in conventional cosmetic products. As examples of the additives, there may favorably be recited, for example, UV absorbers, such as benzophenone derivatives and ethyl 2-cyano-3,3-diphenylacrylate, and so on.

For the cosmetic product for manicure according to the present invention, any ones having configurations known from the past for cosmetic products for manicure may be used without any special respect to the form of the cosmetic product.

While the cosmetic products according to the present invention are detailed above with respect to the general description of cosmetics, make-up cosmetics, cosmetic products for lip rouge, cosmetic products for hair conditioning and cosmetic products for manicure, the cosmetic products according to the present invention are not specifically restricted thereto. The pullulan-cholesterol derivative may be incorporated and used in cosmetic products of any morphological state by compounding in adequate manner. Any kind of cosmetic product can be converted into a cosmetic product exhibiting a characteristic feature not revealed heretofore, by making use of the characteristic feature of the pullulan-cholesterol derivative sufficiently.

The pullulan-cholesterol derivative reveals a feature that it gives birth to a product exhibiting a superior ability of forming a coating film when the proportion of introduction of the cholesteryl groups into the pullulan-cholesterol derivative is in the range from 0.01 to 20 groups per 100 monosaccharide units constituting the pullulan.

When the proportion of introduction of the cholesteryl group exceeds over 20 groups per 100 monosaccharide units, the dissolving performance at room temperature becomes deteriorated.

While the content of the pullulan-cholesterol derivative in the cosmetic product may be different for each specific form of the product, it may in general favorably be in the range from 0.001 to 50 % by weight, preferably from 0.001 to 30 % by weight. When the content of the pullulan-cholesterol derivative is too low, the effect of te present invention will not be obtained, whereas when the content is too high, there may result in occurrence of an adherent touch or a heavy feel of application.

The coating film formed from the pullulan-cholesterol derivative reveals no adherent touch feel and brings about a smooth touch when the cosmetic product has a content of the pullulan-cholesterol derivative of 50 % by weight or less. By utilizing these characteristic features with adequate contrivance in accordance with each specific form of known cosmetic product, it is possible to use the pullulan-cholesterol derivative according to the present invention in every cosmetic product according to the present invention in a favorite manner.

Below, the present invention will further be described in more detail by way of SYNTHESIS EXAMPLES, EXAMPLES and COMPARATIVE EXAMPLES.

### SYNTHESIS EXAMPLE 1

### Synthsis of N-(6-isocyanatohexyl)cholesteryl carbamate

In an eggplant type flask of 1 liter capacity, there were charged 25 grams (0.065 mole) of cholesterol and thereto were added 300 ml of toluene to dissolve it, whereto 17 ml (0.12 mole) of triethylamine were added. Thereto were added 161 grams (0.96 mole) of hexamethylene diisocyanate dissolved in 300 ml of toluene to cause reaction at a temperature of 80 °C for about 6 hours under a nitrogen atmosphere. After termination of the reaction, toluene and the excess of hexamethylene diisocyanate were removed under a reduced pressure. By standing the resulting yellow oily residue at room temperature overnight, pale yellow crystals were formed. The crystals were taken out and thereto was added about 1 liter of hexane, whereupon the mixture was shaken vigorously and, then, the supernatant liquid was removed by decantation. These washing procedures were repeated four times, whereupon the crystals were dried at room temperature for three hours under a reduced pressure, whereby a white solid matter was obtained. A yield amount of 18.25 grams with a yield of 50.9 % was recorded. The observed results of IR are recited below:
IR (KBr, cm⁻¹): 3260, 2320, 1680, 1130

From the above, it was confirmed that N-(6-iso-cyanatohexyl)cholesterylcarbamate was obtained.

### SYNTHESIS EXAMPLE 2

### Synthesis of pullulan-cholesterol derivative (abbreviated hereinafter as CHP 0.9) in which 0.9 cholesteryl group was introduced per 100 monosaccharide units in pullulan

In an eggplant type flask of 1 liter capacity, there were charged 40 grams of pullulan (weight-average molecular weight 108,000) and 420 ml of dimethyl sulfoxide and the mixture was agitated at 80 °C under a nitrogen atmosphere to cause dissolution. Thereto were added 1.78 grams (3.21 millimoles) of N-(6-isocyanatohexyl)cholesterylcarbamate synthesized in Synthesis Example 1 dissolved in 32.4 ml (0.40 mole) of pyridine to cause reaction at 90 °C for 1.5 hours. After termination of the reaction, dimethyl sulfoxide was removed under a reduced pressure, whereupon the resulting oily residue was poured into 6 liters of acetone to cause precipitation.

After removal of the supernatant, 4 liters of acetone were added to the resulting precipitate and the mixture was stood still at room temperature overnight. The precipitate was collected by filtration and was then dried under a reduced pressure. The resulting solid matter was dissolved in dimethyl sulfoxide and the solution was charged in a dialysis membrane (a product of the firm Spectropore with trademark SPECTRA/POR 3; a fractionating molecular weight of 3,500) and was dialyzed against distilled water for one week. 1.5 liters of the resulting polymer solution were subjected to freeze-drying by a conventional procedure, whereby a white solid matter was obtained. The product amounted to 31.7 grams (yield = 76.2 %). The observation results of ¹H-NMR (δ ppm) and of IR are recited below:
¹H-NMR (δ ppm) (DMSO-d₆ /D₂ O = 20/1, vol. TMS):
0.68-2.40, 2.60-4.60, 4.60-5.05
IR (KBr, cm⁻¹): 1680, 1180-900

From integration of the ¹H-NMR spectrum, the surface area for the peak due to cholesteryl group (δ = 0.6 - 2.3) and that due to pullulan (δ = 4.7 - 5.1) were determined, in order to calculate the degree of substitution with cholesteryl group with respect to 100 monosaccharide units. As a result, it was found that the degree of substitution with cholesteryl group per 100 monosaccharide units was 0.9 group. The weight-average molecular weight thereof was determined to be 109,700. From these data, it was confirmed that the compound obtained was CHP 0.9.

### SYNTHESIS EXAMPLE 3

### Synthesis of pullulan-cholesterol derivative (abbreviated hereinafter as CHP 0.1) in which 0.1 cholesteryl group was introduced per 100 monosaccharide units in pullulan

By the same reaction operation as in Synthesis Example 2 with the only exception that the charged amount of N-(6-isocyanatohexyl)cholesterylcarbamate was changed to 0.198 gram (0.357 millimole), CHP 0.1 was synthesized. From integration of the ¹H-NMR spectrum, the surface area for the peak due to cholesteryl group and that due to pullulan were determined, in order to calculate the degree of substitution by cholesteryl group with respect to 100 monosaccharide units. As a result, it was found that the degree of substitution by cholesteryl group per 100 monosaccharide units was 0.1 group. The weight-average molecular weight thereof was determined to be 108,200.

### SYNTHESIS EXAMPLE 4

### Synthesis of pullulan-cholesterol derivative (abbreviated hereinafter as CHP 0.05) in which 0.05 cholesteryl group was introduced per 100 monosaccharide units in pullulan

By the same reaction operation as in Synthesis Example 2 with the only exception that the charged amount of N-(6-isocyanatohexyl)cholesterylcarbamate was changed to 0.099 gram (0.178 millimole), CHP 0.05 was synthesized. From integration of the ¹H-NMR spectrum, the surface area for the peak due to cholesteryl group and that due to pullulan were determined, in order to calculate the degree of substitution by cholesteryl group with respect to 100 monosaccharide units. As a result, it was found that the degree of substitution by cholesteryl group per 100 monosaccharide units was 0.05 group. The weight-average molecular weight thereof was determined to be 108,100.

### SYNTHESIS EXAMPLE 5

### Synthesis of pullulan-cholesterol derivative (abbreviated hereinafter as CHP 10) in which 10 cholesteryl groups were introduced per 100 monosaccharide units in pullulan

By the same reaction operation as in Synthesis Example 2 with the only exception that the charged amount of N-(6-isocyanatohexyl)cholesterylcarbamate was changed to 29.7 grams (53.6 millimoles), CHP 10 was synthesized. From integration of the ¹H-NMR spectrum, the surface area for the peak due to cholesteryl group and that due to pullulan were determined, in order to calculate the degree of substitution by cholesteryl group with respect to 100 monosaccharide units. As a result, it was found that the degree of substitution by cholesteryl group per 100 monosaccharide units was 10 groups. The weight-average molecular weight thereof was determined to be 127,400.

### SYNTHESIS EXAMPLE 6

### Synthesis of pullulan-cholesterol derivative (abbreviated hereinafter as CHP 15) in which 15 cholesteryl groups were introduced per 100 monosaccharide units in pullulan

By the same reaction operation as in Synthesis Example 2 with the only exception that the charged amount of N-(6-isocyanatohexyl)cholesterylcarbamate was changed to 49.5 grams (89.3 millimoles), CHP 15 was synthesized. From integration of the ¹H-NMR spectrum, the surface area for the peak due to cholesteryl group and that due to pullulan were determined, in order to calculate the degree of substitution by cholesteryl group with respect to 100 monosaccharide units. As a result, it was found that the degree of substitution by cholesteryl group per 100 monosaccharide units was 15 groups. The weight-average molecular weight thereof was determined to be 137,000.

### SYNTHESIS EXAMPLE 7 (not part of the present invention)

### Synthesis of manizan-cholesterol derivative (abbreviated hereinafter as CHM) in which 0.9 cholesteryl group was introduced per 100 monosaccharide units in mannan

By the same reaction operation as in Synthesis Example 2 with the exception that 26.2 g of mannan (of a weight-average molecular weight of 85,000) were used instead of pullulan and the charged amounts of N-(6-isocyanatohexyl)cholesterylcarbamate, of pyridine and of dimethyl sulfoxide were changed to 1.08 grams (1.95 millimoles), 19.6 ml and 320 ml, respectively, 21.5 grams of mannan-cholesterol derivative were synthesized. From observations of ¹H-NMR and IR, it was confirmed that the resulting compound was a mannan-cholesterol derivative (CHM). From integration of the ¹H-NMR spectrum, the surface area for the peak due to cholesteryl group and that due to mannan were determined, in order to calculate the degree of substitution by cholesteryl group with respect to 100 monosaccharide units. As a result, it was found that the degree of substitution by cholesteryl group per 100 monosaccharide units was 0.1 group. The weight-average molecular weight thereof was determined to be 85,200.

### EXAMPLE 1

### Preparation of an O/W type milky lotion containing CHP 0.9 and assessment test

Using CHP 0.9 obtained in Synthesis Example 2, an O/W type milky lotion was prepared. For the preparation of the O/W type milky lotion, the components 1 to 10 given below are blended in the proportion as given so that the total amount will sum up to 100 grams. First, components 1 to 3 were blended at 70 °C to cause them to dissolve. After dissolution, the components 4 to 10 were added thereto to cause them to be dispersed. The resulting dispersion was then degassed before being bottled in a predetermined vessel, whereby 100 grams of an O/W type milky lotion containing CHP 0.9 were obtained.

The components used are as given below, wherein the blending proportion is on the weight basis.

| | | |
|---|---|---|
| 1. | Stearic acid | 1 |
| 2. | Beeswax | 2 |
| 3. | Microcrystalline wax | 1 |
| 4. | CHP 0.9 (Synth. Example 2) | 0.3 |
| 5. | Propylene glycol | 5 |
| 6. | Glycerin | 5 |
| 7. | Ethyl alcohol | 2 |
| 8. | Paraoxybenzoic acid ester | 0.3 |
| 9. | Perfume | 0.3 |
| 10. | Pure water | remainder |

Then, assessment tests consisting of a transcription test, a test for the effect of improving artificial rough skin, a test for practical use and a test for sunburn prevention were carried out for assessing the performance of the resulting O/W type milky lotion containing CHP 0.9.

### [Transcription Test]

Two filter papers were provided for, of which one was impregnated with water and the other one was impregnated with squalene. A colorless nylon plate coated with an adequate amount of the CHP 0.9-containing O/W type milky lotion and dried was pressed onto the filter paper impregnated with squalane with ten repeats of up-and-down motions. Also for the filter paper impregnated with water similar operations were performed with repeats of up-and-down motion. After the repetitions of up-and-down motion, the color deepness of the filter paper due to color transference from the nylon plate was visually assessed by one and the same observer. The assessment was realized in a judgement criterion, in which the case where no transcription was recognized scores one point, the case where a scarce transcription was recognized scores two points and the case where transcription was marked scores three points. The assessment test was carried out in five repeats using in each case newly prepared filter papers with impregnation with water and with squalene. The visual assessment of the transcribed color by five repetitions was performed by one and the same person. Each assessment score is given by average of five assessments on the filter papers impregnated with water and with squalene. The results are recited in Table 1.

**Table 1. Transcription test for O/W type milky lotion**

| | Average score |
|---|---|
| Example 1 | 1.6 |
| 2 | 1.8 |
| 3 | 2.0 |
| 4 | 1.6 |
| 5 | 1.8 |
| 6 | 1.6 |
| Comp. Example 1 | 2.4 |
| 2 | 2.6 |
| 3 | 3.0 |
| 4 | 2.6 |

### [Test for the Effect of Improving Artificial Rough Skin]

### «Method of Experiment»

For ten women of ages of 20 - 42, amount of water retained in the horny cell layer, amount of transcutaneous water evaporation and occurrence of efflorescence were observed for assessing the effect of improvement in an artificial rough skin.

First, the skin on the inside of arm was examined by tightly fitting a glass cup having a diameter of 3 cm onto the skin and the cup was charged with 10 ml of 5 % solution of sodium dodecyl sulfate (SDS) while holding the cup fitted on the skin with slight swinging of the solution for 10 minutes, before the solution was collected. Then, on the same skin, a forced rough skin was caused by tightly fitting the cup having a diameter of 3 cm in a similar way onto the skin and the cup was charged with 10 ml of 5 % solution of sodium dodecyl sulfate (SDS) while holding the cup fitted onto the skin with slight swinging of the solution for 20 minutes, before the solution was collected. After one day from the above SDS treatment, each 5 ml of the CHP 0.9-containing O/W type milky lotion were applied onto the treated skin twice a day. The amount of water retained in the horny cell layer was determined in the manner as follows:

### (1) Determination of amount of water retained in the horny cell layer

The amount of water retained in the horny cell layer was determined according to the method for moisture-loaded test for the horny cell layer water proposed by Ueda et al (Flagrance Journal, Extraneous Eddition on 1994 No. 13; "Method for determining moisture-preserving effect of skin") using a high-frequency skin water detector (MODEL SKICON-200, trademark, of the firm IBS).

Determinations were carried out at occasions before the SDS treatment, three days after the SDS treatment, seven days after the SDS treatment and 14 days after the SDS treatment. On the determination, the skin at the site to be examined was washed with a warm water of 37 °C for 30 seconds and five determinations were performed for each person at 20 °C under a relative humidity of 50 %, whereupon average of the determined values was calculated by summing up all the determined values and dividing the sum with the number of persons examined. The average of the whole values was taken at occasions before the SDS treatment, three days after the SDS treatment, seven days after the SDS treatment and 14 days after the SDS treatment. By dividing the average of the whole after each SDS treatment by the average value of the whole before the SDS treatment, the relative values were calculated. The relative values are recited in Table 2. The higher the relative vales are, the greater the amount of water retained in the horny cell layer and, also, the more superior the coated O/W type milky lotion will be

**Table 2. Test for improving artificial rough skin by O/W type milky lotion**

| | Water retained in horny layer | | | Transcutaneous water evaporat. | | | Assessment (score) |
|---|---|---|---|---|---|---|---|
| | Days after the treament | | | | | | |
| | 3 | 7 | 14 | 3 | 7 | 14 | |
| Example | | | | | | | |
| 1 | 1.87 | 1.99 | 2.03 | 0.87 | 0.84 | 0.79 | 0.4 |
| 2 | 1.97 | 2.09 | 2.09 | 0.77 | 0.84 | 0.75 | 0.3 |
| 3 | 2.17 | 2.00 | 1.99 | 0.88 | 0.89 | 0.79 | 0.3 |
| 4 | 2.11 | 1.80 | 2.20 | 0.81 | 0.81 | 0.77 | 0.2 |
| 5 | 1.99 | 1.95 | 2.15 | 0.80 | 0.81 | 0.78 | 0.2 |
| 6 | 1.87 | 1.89 | 1.87 | 0.89 | 0.85 | 0.79 | 1.2 |

| Comp. Example | | | | | | | |
|---|---|---|---|---|---|---|---|
| 1 | 1.11 | 1.00 | 1.04 | 0.96 | 0.94 | 0.97 | 2.1 |
| 2 | 1.07 | 1.00 | 1.04 | 0.97 | 0.94 | 0.99 | 0.4 |
| 3 | 0.87 | 1.09 | 1.00 | 0.96 | 0.94 | 0.98 | 0.2 |
| 4 | 1.07 | 0.89 | 1.03 | 0.97 | 0.95 | 0.99 | 2.2 |

Then, the transcutaneous evapolation was determined in the following manner.

### (2) Determination of transcutaneous evaporation

The transcutaneous evaporation of water from the homey cell layer was determined using a transcutaneous evaporation detector (Model TEWAMETER-TM 200, trademark, supplied from the firm C + K).

Determinations were carried out at occasions before the SDS treatment, three days after the SDS treatment, seven days after the SDS treatment and 14 days after the SDS treatment. On the determination, five determinations were performed for each person directly after the determination of amount of water retained in the horny cell layer of the above (1) and the average values of the determined values for each person were calculated for each person. By summing up all the determined values and dividing the sum with the number of persons examined the average of the whole values was calculated. The average of the whole values was taken at occasions before the SDS treatment, three days after the SDS treatment, seven days after the SDS treatment and 14 days after the SDS treatment. By dividing the average of the whole after each SDS treatment by the average value of the whole before the SDS treatment, the relative values were calculated. The relative values are recited in Table 2. The lower the relative vales are, the greater the amount of water retained in the horny cell layer and, also, the more superior the coated O/W type milky lotion will be.

Then, occurrence of efflorescence was assessed for 10 persons to be examined who have received a coating layer of the CHP 0.9-containing O/W type milky lotion in the above experiments (1) and (2), after all the above experiments have been finished, in accordance with the judgement criterium given below:

### (3) Assessment of efflorescence

The assessment of occurrence of efflorescence was carried out for each person isolately in the attendance of a dermatologist in accordance with the assessment criterium given below:
0 : No dry skin desquamation is recognized.
1 : Slight dry skin desquamation is recognized (slight desquamation or luster).
2 : Clear desquamation of dry skin is recognized with clear discrimination of the border of treated area, clear recognition of exfoliated skin with partial luster and cracks.
3 : Remarkable desquamation of dry skin pieces with clear desquamated dry skin with clear luster and cracks.

Under the assessment critrium given above, ten persons were examined and the results are represented by average value in Table 2. The smaller the numeral values of assessment, the lower the damaging effect on the skin and, thus, more superior the O/W type milk lotion tested will be.

### [Practical Application Test]

### «Method of experiment»

Twenty Japanese women patients of ages of 27 - 42 suffering habitually from rough skin and dry skin were treated by applying the CHP 0.9-containing O/W type milky lotion over a test priod of one month. By oral hearing from each patient, "wet feel", "improvement in stretched skin" and "feel of lubrication" were assessed, in order to examine the beauty skin effect, such as wet feel (water-retentive effect) and improved skin stretching (activating effect) after the one month test as well as the touch feel (feel of lubrication) during the practical application. Number of patients who gave affirmative reply are recited in Table 3.

**Table 3. Practical Application Test with O/W Type Milky Lotion**

| | Affirmative patients among 20 | | | Sunburn prevention |
|---|---|---|---|---|
| | Wet feel | Improved stretch | Lubricat. feel | |
| Example | | | | |
| 1 | 18 | 15 | 18 | 19.0 |
| 2 | 17 | 16 | 18 | 14.5 |
| 3 | 18 | 17 | 18 | 12.2 |
| 4 | 19 | 14 | 17 | 19.4 |
| 5 | 17 | 16 | 15 | 20.0 |
| 6 | 13 | 15 | 13 | 19.6 |

| Comp. Example | | | | |
|---|---|---|---|---|
| 1 | 14 | 11 | 9 | 10.5 |
| 2 | 11 | 10 | 10 | 8.6 |
| 3 | 7 | 9 | 7 | 2.9 |
| 4 | 8 | 11 | 8 | 5.1 |

### [Test for Sunburn Protection]

### «Method of Experiments »

The sunburn protection test was performed by SPF (Sun Protection Factor) determination by animals to assess the sunburn protection effect. Marmots were prepared for the test by depilating back fur using a depilating cream. On this depilated area was coated with the CHP 0.9-containing O/W type milky lotion at a dose of 2 µl/cm². After 15 minutes, the coated area was irradiated by an ultraviolet ray for a definite interval using an ultraviolet lamp (of Toshiba Corp. with trademark of FL-SE). After 24 hours from the irradiation, erythema was examined on the coated area and on the area without coating, wherefrom the least necessary dose of ultraviolet ray for causing a slight erythema was determined. From the calculated least neccesary dose of ultraviolet ray, SPF was calculated. SPF is calculated by dividing the least necessary dose of ultraviolet ray on the coated area by the least necessary dose of ultraviolet ray on the non-coated area. Results for the SPF are recited in Table 3. From the results of the sunburn protection test, it was confirmed that the CHP 0.9-containing O/W type milky lotion was superior in the sunburn protecting effect.

From the above assessment tests, it was proven that the CHP 0.9-containing O/W type milky lotion has marked effects for increasing the water-retentivity in the horny layer (recovery of water retentive function), for decreasing the transcutaneous water evaporation (increase in the moisture barriering function) and for preventing dry efflorescence and is superior not only in the beauty skin effect, such as water-retentivity and activating effect, but also in the sunburn protecting effect, together with superior lubricating feel.

### EXAMPLE 2

### Preparation of CHP 0.1-containing O/W type milky lotion and assessment test

With the same blending proportion and by the same preparation procedures as in Example 1 except that the CHP 0.1 obtained in Synthesis Example 3 was used instead of the CHP 0.9, an O/W type milky lotion was prepared. All the assessment tests were also carried out in the same manner as in Example 1. Results are recited in Tables 1 to 3.

### EXAMPLE 3

### Preparation of CHP 0.05-containing O/W type milky lotion and assessment test

With the same blending proportion and by the same preparation procedures as in Example 1, except that the CHP 0.05 obtained in Synthesis Example 4 was used instead of the CHP 0.9, an O/W type milky lotion was prepared. All the assessment tests were also carried out in the same manner as in Example 1. Results are recited in Tables 1 to 3.

### EXAMPLE 4

### Preparation of CHP 10-containing O/W type milky lotion and assessment test

With the same blending proportion and by the same preparation procedures as in Example 1, except that the CHP 10 obtained in Synthesis Example 5 was used instead of the CHP 0.9, an O/W type milky lotion was prepared. All the assessment tests were also carried out in the same manner as in Example 1. Results are recited in Tables 1 to 3.

### EXAMPLE 5

### Preparation of CHP 15-containing O/W type milky lotion and assessment test

With the same blending proportion and by the same preparation procedures as in Example 1, except that the CHP 15 obtained in Synthesis Example 6 was used instead of the CHP 0.9, an O/W type milky lotion was prepared. All the assessment tests were also carried out in the same manner as in Example 1. Results are recited in Tables 1 to 3.

### EXAMPLE 6

### Preparation of QHM-containing O/W type milky lotion and assessment test

With the same blending proportion and by the same preparation procedures as in Example 1, except that the CHM obtained in Synthesis Example 7 was used instead of the CHP 0.9, an O/W type milky lotion was prepared. All the assessment tests were also carried out in the same manner as in Example 1. Results are recited in Tables 1 to 3.

### SYNTHESIS EXAMPLE 8 (not part of the present invention)

### Synthesis ofpullulan-tristrimethylsiloxysilylpropylcarbamate (Abbreviated hereinafter as TSP) in which 1.7 tristrimethylsiloxysilylpropyl groups were introduced per 100 monosaccharide units in pullulan

Ten grams of a pullulan (having a weight-average molecular weight of 108,000) were dissolved in 300 ml of N-methylpyrrolidone, whereto 0.01 gram of triethylamine was added as the catalyst and thereto was dropped 0.7 gram of tristrimethylsiloxysilylpropyl isocyanate to cause reaction at 100 °C for 2 hours. The reaction mixture was poured into acetone, whereupon the precipitate formed was washed with methanol and was dried. 50 grams of pullulan tristrimethylsiloxysilylpropylcarbamate were obtained. When the degree of substitution by the tristrimethylsiloxysilylpropyl groups per 100 monosaccharide units in the pullulan was calculated from the elementary analysis data, a value of 1.7 groups was obtained. The weight-average molecular weight was found to be 111,000.

### COMPARATIVE EXAMPLE 1

### Preparation of TSP 5-containing O/W type milky lotion and assessment test

With the same blending proportion and by the same preparation procedures as in Example 1, except that the TSP obtained in Synthesis Example 8 was used instead of the CHP 0.9, an O/W type milky lotion was prepared. All the assessment tests were also carried out in the same manner as in Example 1. Results are recited in Tables 1 to 3.

### COMPARATIVE EXAMPLE 2

### Preparation of pullulan-containing O/W type milky lotion and assessment test

With the same blending proportion and by the same preparation procedures as in Example 1, except that a commercial pullulan (weight-average molecular weight of 108,000) was used instead of the CHP 0.9, an O/W type milky lotion was prepared. All the assessment tests were also carried out in the same manner as in Example 1. Results are recited in Tables 1 to 3.

### COMPARATIVE EXAMPLE 3

### Preparation of CHP-not-containing O/W type milky lotion and assessment test

With the same blending proportion and by the same preparation procedures as in Example 1, except that the CHP 0.9 was not incorporated, an O/W type milky lotion was prepared. All the assessment tests were also carried out in the same manner as in Example 1. Results are recited in Tables 1 to 3.

### COMPARATIVE EXAMPLE 4

### Preparation of polyvinyl alcohol-containing O/W type milky lotion and assessment test

With the same blending proportion and by the same preparation procedures as in Example 1, except that a commercial polyvinyl alcohol (a product of Kuraray Co., Ltd. with trademark of KURARE POVAL PVA-224C) was used instead of the CHP 0.9, an O/W type milky lotion was prepared. All the assessment tests were also carried out in the same manner as in Example 1. Results are recited in Tables 1 to 3.

### EXAMPLE 7

### Preparation of 0.001 % CHP containing O/W type milky lotion and assessment test

With the same blending proportion and by the same preparation procedures as in Example 1, except that the blended porportion of CHP 0.9 was changed from 0.3 % by weight to 0.001 % by weight, an O/W type milky lotion was prepared. For the assessment test, only transcription test was carried out and other tests were omitted. Result is recited in Table 4.

**Table 4. Transcription test of O/W milky lotion**

| | Assessment of transcription (score) |
|---|---|
| Example | |
| 7 | 2.6 |
| 8 | 2.2 |
| 9 | 1.0 |
| 10 | 1.2 |

### EXAMPLE 8

### Preparation of 0.01 wt. % CHP-containing O/W milky lotion and assessment test

With the same blending proportion and by the same preparation procedures as in Example 1, except that the blended proportion of CHP 0.9 was changed from 0.3 % by weight to 0.01 % by weight, an O/W type milky lotion was prepared. For the assessment test, only transcription test was carried out and other tests were omitted. Result is recited in Table 4.

### EXAMPLE 9

### Preparation of 50 wt. % CHP-containing O/W milky lotion and assessment test

With the same blending proportion and by the same preparation procedures as in Example 1, except that the blended proportion of CHP 0.9 was changed from 0.3 % by weight to 50 % by weight, an O/W type milky lotion was prepared. For the assessment test, only the transcription test was carried out and other tests were omitted. Result is recited in Table 4.

### EXAMPLE 10

### Preparation of 20 wt. % CHP-containing O/W milky lotion and assessment test

With the same blending proportion and by the same preparation procedures as in Example 1, except that the blended porportion of CHP 0.9 was changed from 0.3 % by weight to 20 % by weight, an O/W type milky lotion was prepared. For the assessment test, only transcription test was carried out and other tests were omitted. Result is recited in Table 4.

### EXAMPLE 11

### Preparation of CHP 0.9-containing beauty wash

A beauty wash was prepared using the CHP 0.9 obtained in Synthesis Example 2. For preparing the beauty wash, the blending components 1 to 8 given below are compounded in the proportion as given, so that the total amount will sum up to 100 grams. First, the components 1 to 4 were mixed at room temperature to attain dissolution. On the other hand, the components 5 to 8 were mixed at room temperature to form a dissolved mixture. To this dissolved mixture, the above-obtained dissolved components 1 to 4 were admixed and agitated to attain dispersion. In this manner, 100 grams of CHP 0.9-containing slightly turbid beauty wash were obtained.

Components blended are recited below with each blending proportion in weight parts:

| | | |
|---|---|---|
| 1. : | CHP 0.9 of Synthesis Example 2 | 0.25 |
| 2. : | Glycerin | 1.5 |
| 3. : | Ethanol | 6 |
| 4. : | Propylene glycol | 1.5 |
| 5. : | Citric acid | 0.01 |
| 6. : | Sodium citrate | 0.1 |
| 7. : | Perfume | 0.05 |
| 8. : | Pure water | remainder |

### EXAMPLE 12

### Preparation of CHP 0.9-containing cream

A cream was prepared using the CHP 0.9 obtained in Synthesis Example 2. For preparing the cream, the blending components 1 to 9 given below are compounded in the proportion as given, so that the total amount will sum up to 100 grams. First, the components 1 to 3 were mixed at 70 °C to attain dissolution. On the other hand, the components 4 to 9 were mixed and were dissolved at 70 °C. To the dissolved mixture of components 1 to 3, the solution of the components 4 to 9 was added and agitated. The mixture was then cooled to 25 °C and was stood still, whereby 100 grams of CHP 0.9-containing white cream were obtained.

Components blended are recited below with each blending proportion in weight parts:

| | | |
|---|---|---|
| 1. : | Squalane | 5 |
| 2. : | 2-ethylhexanoic acid triglyceride | 1 |
| 3. : | Vaseline | 0.5 |
| 4. : | CHP 0.9 (Synthesis Example 2) | 1.5 |
| 5. : | Glycerin | 3 |
| 6. : | 1,3-butanediol | 4 |
| 7. : | Polyglycerolpolyoxybutylene stearyl ether | 2.5 |
| 8. | Perfume | 0.2 |
| 9. | Pure water | remainder |

### EXAMPLE 13

### Preparation of CHP 0.9-containing hair cream

A hair cream was prepared using the CHP 0.9 obtained in Synthesis Example 2. For preparing the hair cream, the blending components 1 to 15 given below are compounded in the proportion as given, so that the total amount will sum up to 100 grams. First, the components 1 to 8 were mixed and dissolved at 70 °C. On the other hand, the components 9 to 15 were mixed and were dissolved at 70 °C. The solution of the components 1 to 8 was added to the solution of the components 9 to 15 and agitated. The mixture was then cooled to 25 °C and was stood still, whereby 100 grams of CHP 0.9 - containing white hair cream were obtained.

Components blended are recited below with each blending proportion in weight parts:

| | | |
|---|---|---|
| 1. : | Squalane | 30.0 |
| 2. : | Vaseline | 3.0 |
| 3. : | Bees wax | 4.0 |
| 4. : | Stearic acid | 4.0 |
| 5. : | Olive oil | 2.0 |
| 6. : | Sorbitan monostearate | 2.5 |
| 7. : | Polyoxyethylenesorbitan mono- | 2.5 |
| | stearate | |
| 8. : | Butylparaben | 0.1 |
| 9. : | CHP 0.9 (Synthesis Example 2) | 3 |
| 10. : | 1,3-butanediol | 2.5 |
| 11. : | Polyethylene glycol 200 | 1.5 |
| 12. : | Triethanolamine | 1.0 |
| 13. : | Paraoxybenzoic acid ester | 0.1 |
| 14. : | Perfume | 0.2 |
| 15. : | Pure water | remainder |

### EXAMPLE 14

### Preparation of CHP 0.9-containing hair lotion

A hair lotion was prepared using the CHP 0.9 obtained in Synthesis Example 2. For preparing the hair lotion, the blending components 1 to 7 given below are compounded in the proportion as given, so that the total amount will sum up to 100 grams. First, the components 1 to 6 were mixed at room temperature to attain dissolution. This solution was admixed to the pure water of component 7 with agitation. In this manner, 100 grams of CHP 0.9-containing slightly turbid liquid hair lotion were obtained.

Components blended are recited below with each blending proportion in weight parts:

| | | |
|---|---|---|
| 1. : | CHP 0.9 (Synthesis Example 2) | 0.1 |
| 2. : | Ethanol | 10 |
| 3. : | Glycerin | 3 |
| 4. : | Carboxymethylchitin | 0.01 |
| 5. : | Vitamin E | 0.1 |
| 6. : | Dyestuff | 0.02 |
| 7. : | Pure water | remainder |

For this CHP 0.9-containing hair lotion, the following test was carried out, in order to examine its hair protecting effect.

### [Test for Assessing Hair Protecting Effect]

### «Method of Experiment»

Ten grams of hair of a Japanese woman who has never experienced any parmanent waving or bleaching treatment were collected as a bundle (of a length of 10 cm) for use as hair sample. Onto this bundle, 3 ml of the CHP 0,9-containing hair lotion were coated and dried in air to subject it to an organoleptic assessment. The organoleptic assessment was performed by five professional panelists of this firm according to the assessment criterion given below to assess smoothness, luster and wet feel (moisture-retentivity) of the hair sample. Average scored points for them are recited in Table 5.

### «Assessment Criterion»

| (Smoothness) | | | | | |
|---|---|---|---|---|---|
| Score | 1 | 2 | 3 | 4 | 5 |
| Assessment | Inferior | Somewhat inferior | Ordinary | Somewhat superior | Superior |

| <Luster> | | | | | |
|---|---|---|---|---|---|
| Score | 1 | 2 | 3 | 4 | 5 |
| Assessment | Inferior | Somewhat inferior | Ordinary | Somewhat superior | Superior |

| (Wet Feel) | | | | | |
|---|---|---|---|---|---|
| Score | 1 | 2 | 3 | 4 | 5 |
| Assessment | Inferior | Somewhat inferior | Ordinary | Somewhat superior | Superior |

**Table 5. Test for assessing effect of hair lotion on hair**

| | Smoothness | Luster | Wet feel |
|---|---|---|---|
| Example | | | |
| 14 | 4.8 | 4.4 | 4.0 |
| 15 | 4.6 | 4.4 | 4.0 |
| 16 | 4.4 | 4.2 | 4.2 |
| 17 | 4.0 | 3.8 | 3.6 |
| 18 | 4.8 | 4.4 | 4.4 |
| 19 | 3.8 | 3.0 | 4.0 |

| Comp. Example | | | |
|---|---|---|---|
| 5 | 3.4 | 3.2 | 3.4 |
| 6 | 3.0 | 2.8 | 3.2 |
| 7 | 2.4 | 2.6 | 3.0 |
| 8 | 3.2 | 1.8 | 2.0 |

As shown in Table 5, it was made clear that the CHP 0.9-containing hair lotion is superior in any of the smoothness, luster and wet feel.

### EXAMPLE 15

### Preparation of CHP 0.1-containing hair lotion and assessment test

With the same blending proportion and by the same preparation procedures as in Example 14 except that the CHP 0.1 obtained in Synthesis Example 3 was used instead of the CHP 0.9, a hair lotion was prepared. All the assessment tests were also carried out in the same manner as in Example 14. Results are recited in Table 5.

### EXAMPLE 16

### Preparation of CHP 0.05-containing hair lotion and assessment test

With the same blending proportion and by the same preparation procedures as in Example 14, except that the CHP 0.05 obtained in Synthesis Example 4 was used instead of the CHP 0.9, a hair lotion was prepared. All the assessment tests were also carried out in the same manner as in Example 14. Results are recited in Table 5.

### EXAMPLE 17

### Preparation of CHP 10-containing hair lotion and assessment test

With the same blending proportion and by the same preparation procedures as in Example 14, except that the CHP 10 obtained in Synthesis Example 5 was used instead of the CHP 0.9, a hair lotion was prepared. All the assessment tests were also carried out in the same manner as in Example 14. Results are recited in Table 5.

### EXAMPLE 18

### Preparation of CHP 15-containing hair lotion and assessment test

With the same blending proportion and by the same preparation procedures as in Example 14, except that the CHP 15 obtained in Synthesis Example 6 was used instead of the CHP 0.9, a hair lotion was prepared. All the assessment tests were also carried out in the same manner as in Example 14. Results are recited in Table 5.

### EXAMPLE 19

### Preparation of CHM-containing O/W type milky lotion and assessment test

With the same blending proportion and by the same preparation procedures as in Example 14, except that the CHM obtained in Synthesis Example 7 was used instead of the CHP 0.9, a hair lotion was prepared. All the assessment tests were also carried out in the same manner as in Example 14. Results are recited in Table 5.

### COMPARATIVE EXAMPLE 5

### Preparation of TSP 5-containing hair lotion and assessment test

With the same blending proportion and by the same preparation procedures as in Example 14, except that the TSP obtained in Synthesis Example 8 was used instead of the CHP 0.9, a hair lotion was prepared. All the assessment tests were also carried out in the same manner as in Example 14. Results are recited in Table 5.

### COMPARATIVE EXAMPLE 6

### Preparation of pullulan-containing hair lotion and assessment test

With the same blending proportion and by the same preparation procedures as in Example 14, except that a commercial pullulan (weight-average molecular weight of 108,000) was used instead of the CHP 0.9, a hair lotion was prepared. All the assessment tests were also carried out in the same manner as in Example 14. Results are recited in Table 5.

### COMPARATIVE EXAMPLE 7

### Preparation of CHP-not-containing hair lotion and assessment test

With the same blending proportion and by the same preparation procedures as in Example 14, except that the CHP 0.9 was not incorporated, a hair lotion was prepared. All the assessment tests were also carried out in the same manner as in Example 14. Results are recited in Table 5.

### COMPARATIVE EXAMPLE 8

### Preparation of polyvinyl alcohol-containing hair lotion and assessment test

With the same blending proportion and by the same preparation procedures as in Example 14, except that a commercial polyvinyl alcohol (a product of Kuraray Co., Ltd. with trademark of KURARE POVAL PVA-224C) was used instead of the CHP 0.9, a hair lotion was prepared. All the assessment tests were also carried out in the same manner as in Example 14. Results are recited in Table 5.

### EXAMPLE 20

### Preparation of CHP 0.9-containing liquid lip rouge and assessment test

69 parts by weight of a polydimethylsiloxane (a product of Shin-Etsu Chemical Co., Ltd. with trademark KF 96, having a standard viscosity of 100,000 mm²/sec) and 15 parts by weight of the CHP 0.9 obtained in Synthesis Example 2 were mixed together with agitation at a temperatrue of 70 - 80 °C to cause dissolution. On the other hand, 5.0 parts by weight of glyceryl triisostearate and 10.0 parts by weight of Red 226 were processed on a roller. This roller-treated mass was admixed to the polydimethylsiloxane solution of CHP 0.9 to disperse it therein. By admixing an adequate amount of perfume to this dispersion after this dispersion had been degassed, 100 grams of a CHP 0.9-containing liquid lip rouge were obtained. In order to assess the properties of this CHP 0.9-containing liquid lip rouge, the following assessment test was carried out.

### [Transcription Test]

Two filter papers were provided for, of which one was impregnated with water and the other one was impregnated with squalene. A colorless nylon plate coated with an adequate amount of the CHP 0.9-containing liquid lip rouge and dried was pressed onto the filter paper impregnated with squalane with ten repeats of up-and-down motions. Also for the filter paper impregnated with water similar operations were performed with repeats of up-and-down motion. After the repetitions of up-and-down motion, the color deepness of the filter paper due to color transference from the nylon plate was visually assessed by one and the same observer (a professional assesser in this firm). The assessment was realized in a judgement criterion, in which the case where no transcription was recognized scores one point, the case where a scarce transcription was recognized scores two points and the case where transcription was remarkable scores three points. The assessment test was carried out in five repeats using in each case newly prepared filter papers with impregnation with water and with squalene. The visual assessment of the transcribed color by five repetitions was performed by one and the same person. Each assessment score is given by an average of five assessments on the filter papers impregnated with water and with squalene. The results are recited in Table 6.

**Table 6. Transcription test for liquid lip rouge**

| | Average score |
|---|---|
| Example 20 | 2.2 |
| 21 | 2.0 |
| 22 | 2.4 |
| 23 | 1.8 |
| 24 | 1.8 |
| 25 | 2.4 |
| Comp. Example 9 | 2.6 |
| 10 | 3.0 |
| 11 | 3.0 |
| 12 | 2.8 |

### EXAMPLE 21

### Preparation of CHP 0.1-containing liquid lip rouge and assessment test

With the same blending proportion and by the same preparation procedures as in Example 20 except that the CHP 0.1 obtained in Synthesis Example 3 was used instead of the CHP 0.9, a liquid lip rouge was prepared. All the assessment tests were also carried out in the same manner as in Example 20. Results are recited in Table 6.

### EXAMPLE 22

### Preparation of CHP 0.05-containing liquid lip rouge and assessment test

With the same blending proportion and by the same preparation procedures as in Example 20, except that the CHP 0.05 obtained in Synthesis Example 4 was used instead of the CHP 0.9, a liquid lip rouge was prepared. All the assessment tests were also carried out in the same manner as in Example 20. Results are recited in Table 6.

### EXAMPLE 23

### Preparation of CHP 10-containing liquid lip rouge and assessment test

With the same blending proportion and by the same preparation procedures as in Example 20, except that the CHP 10 obtained in Synthesis Example 5 was used instead of the CHP 0.9, a liquid lip rouge was prepared. All the assessment tests were also carried out in the same manner as in Example 20. Results are recited in Table 6.

### EXAMPLE 24

### Preparation of CHP 15-containing liquid lip rouge and assessment test

With the same blending proportion and by the same preparation procedures as in Example 20, except that the CHP 15 obtained in Synthesis Example 6 was used instead of the CHP 0.9, a liquid lip rouge was prepared. All the assessment tests were also carried out in the same manner as in Example 20. Results are recited in Table 6.

### EXAMPLE 25

### Preparation of CHM-containing liquid lip rouge and assessment test

With the same blending proportion and by the same preparation procedures as in Example 20, except that the CHM obtained in Synthesis Example 7 was used instead of the CHP 0.9, a liquid lip rouge was prepared. All the assessment tests were also carried out in the same manner as in Example 20. Results are recited in Table 6.

### COMPARATIVE EXAMPLE 9

### Preparation of TSP 5-containing liquid lip rouge and assessment test

with the same blending proportion and by the same preparation procedures as in Example 20, except that the TSP obtained in Synthesis Example 8 was used instead of the CHP 0.9, a liquid lip rouge was prepared. All the assessment tests were also carried out in the same manner as in Example 20. Results are recited in Table 6.

### COMPARATIVE EXAMPLE 10

### Preparation of pullulan-containing liquid lip rouge and assessment test

With the same blending proportion and by the same preparation procedures as in Example 20, except that a commercial pullulan (weight-average molecular weight of 108,000) was used instead of the CHP 0.9, a liquid lip rouge was prepared. All the assessment tests were also carried out in the same manner as in Example 20. Results are recited in Table 6.

### COMPARATIVE EXAMPLE 11

### Preparation of CHP-not-containing liquid lip rouge and assessment test

With the same blending proportion and by the same preparation procedures as in Example 20, except that the CHP 0.9 was not incorporated, a liquid lip rouge was prepared. A11 the assessment tests were also carried out in the same manner as in Example 20. Results are recited in Table 6.

### COMPARATIVE EXAMPLE 12

### Preparation of polyvinyl alcohol-containing liquid lip rouge and assessment test

With the same blending proportion and by the same preparation procedures as in Example 20, except that a commercial polyvinyl alcohol (a product of Kuraray Co., Ltd. with trademark of KURARE POVAL PVA-224C) was used instead of the CHP 0.9, a liquid lip rouge was prepared. All the assessment tests were also carried out in the same manner as in Example 20. Results are recited in Table 6.

### EXAMPLE 26

### Preparation of CHP 0.9-containing colored manicure and assessment test

10.82 parts by weight of nitrocellulose, 9.74 parts by weight of a toluenesulfonamide formaldehyde resin (a product of the firm Akuzo with trade mark KETJENFLEX MS 80), 6.495 parts by weight of tributyl acetylcitrate (a product of Pfizer K.K. with trademark CITROFLEX A4), 30.91 parts by weight of toluene, 21.64 parts by weight of butyl acetate, 9.27 parts by weight of ethyl acetate, 7.72 parts by weight of isopropyl alcohol, 1.35 parts by weight of stearalkonium hectorite, 1.00 part by weight of the CHP 0.9 obtained in Synthesis Example 2 and 0.055 part by weight of citric acid were mixed to cause dissolution, whereby a total of 100 grams of a colored manicure liquid was obtained. In order to evaluate the properties of this colored manicure liquid, assessments given below were carried out.

### «Assessment of adhesiveness»

The CHP 0.9-containing colored manicure liquid was tested by practical use by seven ordinary women of average age of 23.2 for one week. After such practical use, each volunteer was asked by a professional investigator individually for her judgement as to the adhesiveness. The case where the volunteer gave an answer that the adhesiveness was better scores three points. The case where the volunteer gave an answer that the adhesiveness was not worse nor better scores two points. The case where the volunteer gave an answer that the adhesiveness was worse scores one point. All the answers were summarized. The results are recited in Table 7.

### «Assessment of luster»

The CHP 0.9-containing colored manicure liquid was tested by practical use by seven ordinary women of average age of 23.2 for one week. After such practical use, each volunteer was asked by a professional investigator individually for her judgement as to the luster. The case where the volunteer gave an answer that the luster was better scores three points. The case where the volunteer gave an answer that the luster was not worse nor better scores two points. The case where the volunteer gave an answer that the luster was worse scores one point. All the answers were summarized. The results are recited in Table 7.

**Table 7. Test of assessment of colored manicure liquid**

| | Adhesiveness (total score summed) | Luster (total score summed) |
|---|---|---|
| Example | | |
| 26 | 17 | 16 |
| 27 | 15 | 18 |
| 28 | 13 | 15 |
| 29 | 19 | 16 |
| 30 | 18 | 14 |
| 31 | 14 | 15 |

| Comp. Example | | |
|---|---|---|
| 13 | 11 | 14 |
| 14 | 12 | 7 |
| 15 | 9 | 8 |
| 16 | 11 | 10 |

### EXAMPLE 27

### Preparation of CHP 0.1-containing colored manicure liquid and assessment test

With the same blending proportion and by the same preparation procedures as in Example 26 except that the CHP 0.1 obtained in Synthesis Example 3 was used instead of the CHP 0.9, a colored manucure liquid was prepared. All the assessment tests were also carried out in the same manner as in Example 26. Results are recited in Table 7.

### EXAMPLE 28

### Preparation of CHP 0.05-containing colored manicure liquid and assessment test

With the same blending proportion and by the same preparation procedures as in Example 26, except that the CHP 0.05 obtained in Synthesis Example 4 was used instead of the CHP 0.9, a colored manicure liquid was prepared. All the assessment tests were also carried out in the same manner as in Example 26. Results are recited in Table 7.

### EXAMPLE 29

### Preparation of CHP 10-containing colored manicure liquid and assessment test

With the same blending proportion and by the same preparation procedures as in Example 26, except that the CHP 10 obtained in Synthesis Example 5 was used instead of the CHP 0.9, a colored manicure liquid was prepared. All the assessment tests were also carried out in the same manner as in Example 26. Results are recited in Table 7.

### EXAMPLE 30

### Preparation of CHP 15-containing colored manicure liquid and assessment test

With the same blending proportion and by the same preparation procedures as in Example 26, except that the CHP 15 obtained in Synthesis Example 6 was used instead of the CHP 0.9, a colored manicure liquid was prepared. All the assessment tests were also carried out in the same manner as in Example 26.

### Results are recited in Table 7.

### EXAMPLE 31 (not part of the present invention)

### Preparation of CHM-containing colored manicure liquid and assessment test

With the same blending proportion and by the same preparation procedures as in Example 26, except that the CHM obtained in Synthesis Example 7 was used instead of the CHP 0.9, a colored manicure liquid was prepared. All the assessment tests were also carried out in the same manner as in Example 26. Results are recited in Table 7.

### COMPARATIVE EXAMPLE 13

### Preparation of TSP 5-containing colored manicure liquid and assessment test

With the same blending proportion and by the same preparation procedures as in Example 26, except that the TSP obtained in Synthesis Example 8 was used instead of the CHP 0.9, a colored manicure liquid was prepared. All the assessment tests were also carried out in the same manner as in Example 26. Results are recited in Table 7.

### COMPARATIVE EXAMPLE 14

### Preparation of pullulan-containing colored manicure liquid and assessment test

With the same blending proportion and by the same preparation procedures as in Example 26, except that a commercial pullulan (weight-average molecular weight of 108,000) was used instead of the CHP 0.9, a colored manicure liquid was prepared. All the assessment tests were also carried out in the same manner as in Example 26. Results are recited in Table 7.

### COMPARATIVE EXAMPLE 15

### Preparation of CHP-not-containing colored manicure liquid and assessment test

With the same blending proportion and by the same preparation procedures as in Example 26, except that the CHP 0.9 was not incorporated, a colored manicure liquid was prepared. All the assessment tests were also carried out in the same manner as in Example 26. Results are recited in Table 7.

### COMPARATIVE EXAMPLE 16

### Preparation of polyvinyl alcohol-containing manicure liquid and assessment test

With the same blending proportion and by the same preparation procedures as in Example 26, except that a commercial polyvinyl alcohol (a product of Kuraray Co., Ltd. with trademark of KURARE POVAL PVA-224C) was used instead of the CHP 0.9, a colored manicure liquid was prepared. All the assessment tests were also carried out in the same manner as in Example 26. Results are recited in Table 7.

As decribed above, it is possible according to the present invention to provide novel cosmetic products which are superior markedly in the beauty skin effect and in the beauty hair effect due to moisture-retentive function, rough skin preventing effect, coating film-forming ability and so on and which does not suffer from any safety problem with an adaptively maintained moisture preserving ability and coating film-formation ability.

### INDUSTRIAL APPLICABILITY

The cosmetic products according to the present invention can be used adaptively as, for example, cosmetic product for skin care, cosmetic product for hair conditioning and cosmetic product for make-up.

## Claims

1. Cosmetic use of a pullulan-cholesterol derivative in a cosmetic product,
wherein the hydroxyl groups of the monosaccharide units constituting the pullulan-cholesterol derivative are substituted in a proportion of 0.01 to 20 groups per 100 monosaccharide units, by a radical represented by the formula (1) 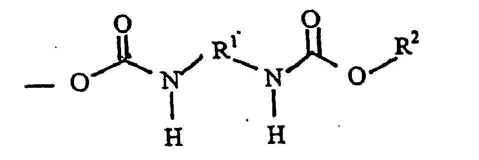 in which R¹ denotes a hydrocarbyl of 1-10 carbon atoms and R² represents a cholesteryl group, as a moisture retaining agent and/or as a film-forming agent.

2. The cosmetic use as claimed in claim 1, wherein the proportion of introduction of cholesteryl groups by substituting for the hydroxyl groups of the monosaccharide units constituting pullan is 0.05-1.5 groups per 100 monosaccharide units.

3. The cosmetic use as claimed in claim 2, wherein the proportion of introduction of cholesteryl groups by substituting for the hydroxyl groups of the monosaccharide units constituting pullan is 0.1-10 groups per 100 monosaccharide units.

4. The cosmetic use as claimed in any one of claims 1 to 3, wherein the content of the pullan-cholesterol derivative is in the range from 0.001 to 50 %, based on the total weight of the cosmetic product.

5. The cosmetic use as claimed in any one of claims 1 to 4, wherein the cosmetic product is a skin care cosmetic, make-up cosmetic or hair conditioning cosmetic.

6. The cosmetic use as claimed in any one of claims 1 to 4, wherein the cosmetic product is an emulsion, a beauty wash, a rouge, a manicure or a hair lotion.

## Patentansprüche

1. Kosmetische Verwendung eines Pullulan-Cholesterinderivats als Feuchtigkeit zurückhaltendes Mittel und/oder als filmbildendes Mittel in einem Kosmetikprodukt,
wobei die Hydroxygruppen der Monosaccharideinheiten des Pullulan-Cholesterinderivats in einem Verhältnis von 0,01 bis 20 Gruppen pro 100 Monosaccharideinheiten durch einen Rest der Formel (1) substituiert sind, 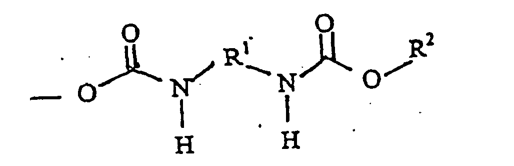 wobei R¹ ein Kohlenwasserstoffrest mit 1-10 Kohlenstoffatomen und R² eine Cholesterylgruppe ist.

2. Kosmetische Verwendung nach Anspruch 1, wobei das Einführungsverhältnis der Cholesterylgruppen durch Substitution der Hydroxygruppen der Pullulanmonosaccharideinheiten 0,05-15 Gruppen pro 100 Monosaccharideinheiten beträgt.

3. Kosmetische Verwendung nach Anspruch 2, wobei das Einführungsverhältnis der Cholesterylgruppen durch Substitution der Hydroxygruppen der Pullulanmonosaccharideinheiten 0,1-10 Gruppen pro 100 Monosaccharideinheiten beträgt.

4. Kosmetische Verwendung nach einem der Ansprüche 1 bis 3, wobei der Gehalt des Pullulan-Cholesterinderivats im Bereich von 0,001 und 50% liegt, bezogen auf das Gesamtgewicht des Kosmetikprodukts.

5. Kosmetische Verwendung nach einem der Ansprüche 1 bis 4, wobei das Kosmetikprodukt ein Hautpflegemittel, ein Make-up oder ein Haarpflegemittel ist.

6. Kosmetische Verwendung nach einem der Ansprüche 1 bis 4, wobei das Kosmetikprodukt eine Emulsion, ein Gesichtswasser, ein Lippenstift, ein Nagellack oder eine Haarlotion ist.

## Revendications

1. Utilisation cosmétique d'un dérivé de pullulane-cholestérol dans un produit cosmétique,
dans laquelle les groupes hydroxyle des unités de monosaccharide constituant le dérivé de pullulane-cholestérol sont substitués dans une proportion de 0,01 à 20 groupes pour 100 unités de monosaccharide, par un radical représenté par la formule (1) 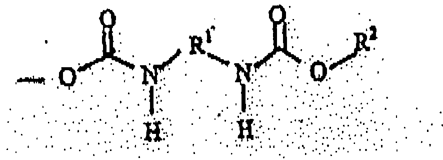 dans laquelle R¹ représente un hydrocarbyle ayant 1-10 atomes de carbone et R¹ représente un groupe cholestéryle, comme agent de rétention d'humidité et/ou comme agent filmogène.

2. Utilisation cosmétique selon la revendication 1, dans laquelle la proportion d'introduction de groupes cholestéryle par substitution des groupes hydroxyle des unités de monosaccharide constituant le pullulane est do 0,05-15 groupes pour 100 unités de monosaccharides.

3. Utilisation cosmétique selon la revendication 2, dans laquelle la proportion d'introduction de groupes cholestéryle par substitution des groupes hydroxyle des unités de monosaccharide constituant le pullulane est de 0,1-10 groupes pour 100 unités de monosaccharides.

4. Utilisation cosmétique selon l'une quelconque des revendications 1 à 3, dans laquelle la teneur en dérive de pullulane-cholestérol est dans la gamme comprise entre 0,001 et 50%, rapporté au poids total du produit cosmétique.

5. Utilisation cosmétique selon l'une quelconque des revendications 1 à 4, dans laquelle le produit cosmétique est un produit cosmétique pour les soins de la peau, un produit cosmétique de maquillage ou un produit cosmétique de conditionnement des cheveux.

6. Utilisation cosmétique selon l'une quelconque des revendications 1 à 4, dans laquelle le produit cosmétique est une émulsion, un soin do beauté, un rouge à lèvres, un produit de manucure ou une lotion capillaire.
